Europäisches Patentamt

(19) European Patent Office

Office européen des brevets

(11) Publication number : **0 508 809 A1**

# (12) EUROPEAN PATENT APPLICATION

(21) Application number : **92303223.9**

(22) Date of filing : **10.04.92**

(51) Int. Cl.⁵ : **C12N 7/04,** C12N 15/86,
C12N 15/12, C12N 15/41,
C12N 15/47, C12N 15/49,
C12N 15/87, C12N 5/10,
C12N 15/85, A61K 37/00

(30) Priority : **10.04.91 GB 9107631**

(43) Date of publication of application :
**14.10.92 Bulletin 92/42**

(84) Designated Contracting States :
**AT BE CH DE DK ES FR GB GR IT LI LU NL PT SE**

(71) Applicant : **BRITISH BIO-TECHNOLOGY LIMITED**
**Watlington Road**
**Cowley Oxford OX4 5LY (GB)**

(72) Inventor : **Adams, Sally Elizabeth**
**British Bio-technology Limited, Watlington Road**
**Cowley, Oxford OX4 5LY (GB)**
Inventor : **Burns, Nigel Robert**
**British Bio-technology Limited, Watlington Road**
**Cowley, Oxford OX4 5LY (GB)**
Inventor : **French, Timothy John**
**British Bio-technology Limited, Watlington Road**
**Cowley, Oxford OX4 5LY (GB)**
Inventor : **Gearing, Andrew John Hubert**
**British Bio-technology Limited, Watlington Road**
**Cowley, Oxford OX4 5LY (GB)**
Inventor : **Kingsman, Alan John**
**British Bio-technology Limited, Watlington Road**
**Cowley, Oxford OX4 5LY (GB)**
Inventor : **Kingsman, Susan Mary**
**British Bio-technology Limited, Watlington Road**
**Cowley, Oxford OX4 5LY (GB)**

(74) Representative : **Sheard, Andrew Gregory et al**
**Kilburn & Strode 30, John Street**
**London WC1N 2DD (GB)**

(54) **Proteinaceous lipid-containing particles.**

(57) Proteinaceous, lipid-containing particles can be prepared by co-expressing in a host cell (i) a self-assembling protein moiety in circumstances where the protein assembles to form a core, which then buds off from the host cell, thereby acquiring a lipid envelope derived from the host cell membrane and (ii) a membrane-bound protein moiety, which becomes integrated in the lipid envelope. The particles have a wide variety of uses. They may be used as an antigen presentation system, or they may for example have site-specific targeting ability, fusogenic properties, enzymic activity, cytotoxic activity, diagnostic utility and/or pharmaceutical activity.

EP 0 508 809 A1

# FIG . 1
## Construction of HIV-1 GAG cassette

This invention relates to proteinaceous, lipid- containing particles. Proteinaceous particles have previously been proposed in the preparation of vaccines. Correct presentation of an antigen to an animal's immune system is a key requirement for an effective sub-unit vaccine or immunogen. The assembly of the antigenic determinants into a high molecular weight complex or particle appears to be significant in enhancing an effective immune response.

Prior to the present invention a number of self- assembling antigen presentation systems have been developed. These include systems based on fusion of antigens to the Hepatitis core antigen (Beesly *et al*, l990 *Bio/Technology* **8**, 644-649), Tobacco mosaic virus coat protein (Haynes *et al*, l986, *Bio/Technology* **4**, 637), poliovirus (Burke *et al*, l988 *Nature* **332**, 8l-82) and yeast retrotransposon Tyl pl (WO-A-8803563 and WO-A-8803562). All of these systems form multimeric fusion protein complexes. However all of them involve a single protein, which assembles into particles intracellularly. Therefore, none of these particulate structures is enveloped within a lipid bilayer. Consequently, these systems do not offer the opportunity to modify the immune responses to the particles by conferring specific properties upon such a bilayer.

A prior self-assembling particulate antigen presentation system which includes a lipid membrane component is based on the Hepatitis B surface antigen (Valenzuela *et al*, l985, *Bio/Technology* **3**, 323). However, the membrane, which is an integral part of these particles, is formed as a by-product of the manufacturing process. Therefore, it is difficult to regulate the insertion of specific membrane bound proteins into the lipid component of these particles during the manufacturing process. When two related viruses infect the same cell the process of phenotypic mixing may occur. This process occurs when the progeny virions contain the genome of one virus, but the structural proteins of either, or both, viruses. Such phenotypic mixing has been observed between Vesicular Stomatitis virus (VSV) and a number of retroviruses, whereby the reciprocal exchange of envelope proteins occurs; in other words, both VSV particles bearing retroviral membrane glycoproteins and retroviral proteins bearing VSV glycoproteins can be detected in mixed viral stocks (see Weiss in "RNA Tumor Viruses", Cold Spring Harbor Laboratory, l984, pp209-260). Phenotypic mixing has also been observed between retroviruses, one of which may be replication-defective (Weiss *ibid*). Whilst such phenotypically mixed virions can be considered as multivalent particulate carriers bearing a heterologous membrane protein, they have a number of disadvantages: (l) all such preparations a priori contain infectious retrovirus, which makes them unsuitable for therapeutic use; (2) inactivation of the active virus may destroy the biological activity of the protein and the antigenicity of the epitopes; (3) the requirement for infectious retrovirions for the production of phenotypically mixed virions means that the heterologous protein must act as a viral receptor, thereby restricting the range of proteins that can be incorporated into such particles; and (4) the production process for the manufacture of such particles would be highly expensive and difficult to control.

Haffar *et al* (J. Virol. **64**(6) 2653-2659 (l990)) have reported HIV gag-env particles containing viral RNA produced in a vaccinia expression system in African green monkey kidney cells. As expected, since the env protein was homologous with the gag protein, the two proteins were associated in the particles produced.

Vzoror *et al* (*AIDS Research and Human Retroviruses* **7**(l) 29-36 (l99l)) similarly produced particles, using a vaccinia expression system, in which HIV env proteins were present with homologous gag proteins.

Young *et al*, in *Science* **250** l422 ( l990) have described the incorporation of heterologous and chimeric proteins into avian leukosis virus virions (ALV). Specifically, the protein CD4 and chimeras comprised of the extracellular domain of CD4 fused to the transmembrane and cytoplasmic domains of ALV or MLV transmembrane ENV proteins, expressed via recombinant DNA technology in susceptible cells, were shown to incorporate into replication competent ALV virions. Although this system has the advantage that, in principle, any membrane bound protein could be incorporated into an ALV particle, it also relies on the use of live, infectious retrovirus; in addition, if a live retrovirus with heterologous membrane protein is used, then there is the possibility of recombination, thereby producing a novel retrovirus with a novel cell tropism. Consequently the system still suffers from the other disadvantages referred to above.

There is therefore a problem in preparing proteinaceous, lipid-containing particles, which may be useful inter alia in the preparation of vaccines, without encountering the difficulties referred to above, particularly the requirement of using replication-competent retroviruses. The present invention relates to a solution to this problem.

It has been found that proteinaceous, lipid-containing particles can be prepared from budding proteins. Such proteins can be prepared by recombinant DNA technology in a host cell, without the presence of infectious viral nucleic acid, and can become at least partially enveloped by lipid as they bud off from the host cell.

According to a first aspect of the invention, there is provided a proteinaceous, lipid-containing particle comprising a budding assembly formed from a plurality of self-assembling protein moieties, the assembly being at least partially enveloped with a lipid bilayer, and one or more membrane-bound protein moieties, heterologous with respect to the self-assembling protein moieties, wherein, if the self-assembling protein moieties are of viral origin, the particle is free of infectious viral nucleic acid. Particles in accordance with the invention are synthetic.

The invention therefore does not for example extend either to natural viruses or to natural phenotypically mixed viruses. Particles in accordance with the invention may be distinguished from such natural viruses in that they do not contain all the components of infectious viruses, whether phenotypically mixed or not. For example, infectious retroviral nucleic acid will usually be missing. The invention therefore avoids the use of replication-competent retroviruses.

A reference to a "protein" in this specification includes a reference to a primary expression product and, where appropriate, a reference to a post-translationally modified (for example glycosylated) protein.

The invention solves the problem of producing an antigen presentation system. However, it does more than this. Particles in accordance with the invention have a wide variety of uses and applications, as will be discussed later.

In an important embodiment of the invention, the self-assembling protein moieties will simply be self-assembling protein molecules. The following description refers primarily to this embodiment, but should be understood to apply additionally to other embodiments, as will subsequently be made clear.

A first component of a particle in accordance with the invention is a budding assembly formed from a plurality of self-assembling protein molecules. The protein molecules are capable of assembling into particles, which then have the ability to acquire a lipid membrane on budding from the cell in which they were assembled and, usually, synthesised. Although in principle the protein molecules may be derived from any source, it is preferred for them to be of viral origin (or at least to be related to proteins of viral origin). Many if not all viruses include protein molecules capable of assembling into particles. It is preferred however for the self-assembling protein to be a GAG protein of a retrovirus. For example, the budding core molecule of Human Immunodeficiency Virus (HIV) may be suitable. The budding assembly can be referred to as a "core" assembly or "core", as it forms the core of particles of the present invention; it should not be taken, however, to be necessarily the same as, or even derived from, a viral core protein.

Preferred GAG proteins include those of retroviruses HIV- I, HIV-2, HTLV-I, HTLV-II, HTLV-III, (ie including T-lymphocyte viruses) SIV, BIV, EIAV, CIAV, Murine Leukaemia Virus, Moloney Murine Leukaemia Virus and Feline Leukaemia Virus. The most preferred proteins are viral core proteins, including the GAG proteins from the retroviruses Human Immunodeficiency Virus (HIV) (Gheysen *et al*, l989, *Cell* **59**, l03-ll2), Simian Immunodeficiency Virus (SIV) (Delchambre *et al*, l989 *EMBO J.* **8**, 2653-2660) and Bovine Immunodeficiency-like Virus (BIV) (Rasmussen *et al*, l990 *Virology* **l78** 435-45l).

It has been shown that immune responses to the GAG proteins of retroviruses has a significant role in the onset and progression of retrovirus induced disease. Therefore, a GAG protein in a particle of this invention will also act as an important immunogenic component of an antigen presentation system, which is one of the uses to which the invention may be put.

The self-assembling protein does not have to be viral or derived from a virus. For example it may be, or be derived from, the Ty protein of a yeast retrotransposon or any other retrotransposable elements such as the copia-like elements of insects.

Self-assembling protein molecules useful in the present invention may be the same as natural self-assembling molecules. However, they do not have to be; for example, the self-assembling sequence of a naturally self-assembling protein molecule may be supplemented by one or more additional amino acids which need not contribute to the self-assembling ability (although they may do); the additional amino acid(s) may for example contribute to antigenicity. Alternatively or in addition, the sequence of a naturally self-assembling protein molecule may be modified for other purposes by amino acid substitution, deletion and/or addition. In some embodiments of the invention the nucleic acid binding ability of the protein (particularly if it is of viral origin) may be modified. Such a modification may have the effect of reducing or even eliminating the protein's nucleic acid binding activity, for example to help ensure that little or no viral nucleic acid is incorporated in the particle; equally, however, the nucleic acid binding characteristics may simply be modified, rather than reduced, for example so that the particle may be used as a delivery system for nucleic acid with which the protein is not normally associated. Such nucleic acid includes antisense RNA, RNA with digesting or cleaving activity, such as ribozymes, and DNA and mRNA coding for a biologically active peptide which can be released on site in a target cell; the present invention can therefore be used for gene therapy.

Modified or natural GAG proteins are among those preferred for use in the invention.

When a self-assembling protein molecule contains a self-assembling sequence and additional amino acids, it may be regarded as comprising a first amino acid sequence and a second amino acid sequence. The first amino acid sequence is responsible for conferring on the protein molecule its self-assembling property (and usually the ability of the assembly to bud) and the second amino acid sequence may confer some desired biological activity. Examples of biological activities of the second amino acid sequence include antigenicity, for the generation of a specific immune response to a pathological agent, enzymic activity and cytotoxic activity. The second amino acid sequence may confer upon the particle antigenicity against any protein derived from a pathological agent, including proteins of viral, bacterial, fungal and protozoal origin. Ideally the second amino acid sequence will be substantially homologous to a sequence which has been demonstrated to play a significant role in generating an immune response which reduces or prevents the onset or progression of disease, such as (for example viral) infectious disease. For instance, the third variable domain of HIV-I gpl20 has been demonstrated to be the dominant neutralising determinant of HIV-I. Therefore, a preferred second amino acid sequence would be substantially homologous to the third variable domain of HIV-I gpl20.

However the self-assembling protein molecule is modified (if at all) it, or at least a self-assembling part of it, will generally have at least 70 %, and preferably at lest 80%, 90% or even 95% homology with a natural protein. Nucleic acid coding for the self-assembling protein may hybridise with nucleic acid coding for a natural self-assembling protein (or would do so but for the degeneracy of the genetic code), for example under stringent conditions (such as at approximately 35°C to 65°C in a salt solution of approximately 0.9 molar).

The self-assembling protein molecules used in the core assembly of particles in accordance with the invention will usually be all the same as each other. Different protein molecules can however be used as long as they are sufficiently compatible with each other so that the particle-forming ability is not lost.

Particles in accordance with the invention also contain a lipid bilayer at least partially enveloping the core assembly of self-assembling proteins. The lipid bilayer will generally be derived from a membrane (usually the plasma membrane, but possibly the endoplasmic reticulum, the golgi apparatus or the nuclear membranes of the cell in which the assembly is put together and/or in which the synthesis of the self- assembling proteins has taken place. Its nature is not believed to be particularly critical, and so particles in accordance with the invention can be produced in a wide variety of cells, as will be discussed below. As a generality, however, it is true to say that natural lipid bilayers comprise a bilayer of a group of phospholipids and this will tend to be influence the nature of the lipid in the particles of the present invention. The presence of the lipid may well confer greater and/or broader immunogenic properties on the particle than would be the case if it consisted of self-assembling proteins alone.

It is preferred that the lipid substantially completely envelope the core assembly in a particle in accordance with the invention. Some deviation from this preference is tolerated, and may be advantageous if for example protein protrudes through the lipid envelope. Such discontinuities as there may be, however, will not usually be substantial. A particle comprising a core assembly flecked with isolated patches of lipid is not preferred.

As discussed above, the self-assembling protein molecules may be modified to mediate various biological activities. Particles according to the invention, however, have a great advantage in that for even greater flexibility, they incorporate, as a third component, membrane-bound moieties in the lipid which at least partially envelopes the core assembly. The membrane-bound moiety will comprise (that is to say will consist of or include) a membrane- (for example a plasma membrane-) localisation and/or retention signal. Such a signal will generally be a sequence of amino acids so chosen (whether by man or nature) in terms of properties and order to have an affinity with the lipid component of the particles.

The membrane-bound moiety can be fused to the particle-forming moiety, which is one reason why both these components have been referred to as moieties, rather than separate proteins, in the invention at its broadest. However, in preferred embodiments of the invention, the particle-forming moiety and the membrane-bound moiety are separate proteins. The following discussion of the membrane-bound moiety refers primarily to the case when it is a separate molecule, but it should be understood that the discussion applies anaogously to the alternative case.

As the membrane-bound protein is heterologous with respect to the self-assembling protein, the invention is directed to particles other than those containing a natural self-assembling protein and its naturally associated natural membrane-bound protein. Particles including self-assembling proteins and membrane-bound proteins derived from the same source, but wherein one or more of the proteins is modified from the natural material, are also less preferred in some embodiments.

Preferred membrane-bound proteins confer additional biological properties upon the particles. These properties include: conferring an immunogenic ability (or improved or altered immunogenic ability) on the membrane protein; a site-specific targeting ability; fusogenic properties; binding or packaging activity; enzymic activity; cytotoxicity; pharmaceutical activity; and/or nucleic acid-binding activity.

If the membrane bound protein is included because of its immunological properties, then it may be, or include sequence from, a protein from any pathogenic organism, including viruses, bacteria, fungi and protozoa. Preferred membrane bound proteins include those of (or homologous with) viruses, including the influenza virus haemagglutinin protein and the env-derived proteins of retroviruses, including HIV- I.

In other preferred embodiments, the membrane bound protein will confer upon the particle the property of binding to specified cells or molecules. The cells may be of the immune system and may include antigen- presenting cells (APCs). In this configuration it is expected an enhanced immune response, both humoral and cellular, will be generated by a given dose of antigen, because a greater proportion of the administered material will be taken up by the cells of the immune system. When included for this reason, preferred proteins include CD4, CD8, molecules involved in leukocyte-endothelium and/or leukocyte-leukocyte interaction such as ICAM-I, ICAM-2, VCAM and the adjuvanting/targeting molecule BBI/B7 (the ligand or CD28), as well as antibody molecules, or other specific binding molecules, such as active fragments of antibody molecules, which have specificities for cells of the immune system. Other suitable proteins may be of viral origin such as gpl60 of HIV-I, which binds to the T-cell ligand, CD4.

Other cell types may also be targeted via the membrane-bound protein. For example, influenza haemagglutinin will target the particles to cells bearing sialic acid on their surface. Ligands to tumour-specific antigens will target the particles to the appropriate tumour.

In addition to or instead of being targeted to cells, membrane-bound protein may be targeted to specific molecules such as antibodies or receptors or even to viruses such as retroviruses.

Preferred fusogenic molecules include those of viral origin such as those containing some or all of the sequence(s) of HIV-I gp4I, influenza haemagglutinin, Vesicular Stomatitis Virus G Protein (VSV-G), Sendai Virus fusion factor (F), and the glycoproteins of Semliki Forest Virus. It is expected that particles without fusogenic activity will be taken up by endocytosis and become incorporated within lysosomes. By conferring fusogenic properties upon the particles the particle forming protein will be delivered directly into the cytoplasm. Direct delivery into the cytoplasm is expected to alter the spectrum of the immune response generated to the proteins comprising the particles. If the particle forming protein is a fusion protein of an enzyme or toxin, then delivery into the cytoplasm will prevent degradation of the enzymic or toxic activity by the lysosomes. Likewise, the delivery of a biologically active RNA will enable lysozymes to be avoided.

In certain embodiments, the particle acquires the membrane-bound protein in its native configuration during budding from the cells in which the particle forming protein and membrane bound protein are co-expressed. In other embodiments the membrane-bound protein can be modified to encode at its C-terminus sequences which will enhance the interaction between the membrane bound protein and the particle forming protein. For example, the membrane and/or cytoplasmic domain of the membrane bound protein would be altered, via recombinant DNA technology, to the sequence of the membrane and/or cytoplasmic domain of the viral envelope protein from which the particle forming protein is also derived. There is often a specific interaction between the retroviral GAG proteins and their cognate envelope proteins and it is expected that this interaction will be a property of the chimeric membrane protein. Such an interaction will lead to more efficient incorporation of the membrane bound protein into the particles. For example, if the particle-forming protein is the GAG protein of HIV-I and the membrane bound protein is to have at least some of the properties of VCAM, then it may be advantageous to replace the C-terminus of VCAM with the C-terminus of HIV-I gp4I.

Membrane-bound protein moieties useful in the invention may be the same as natural proteins or modified with respect to natural proteins.

However the membrane-bound protein moiety is modified (if at all) it, or at least a membrane-binding part of it, will generally have at least 70 %, and preferably at least 80 %, 90% or even 95% homology with a natural protein. Nucleic acid coding for the membrane-binding protein may hybridise with nucleic acid coding for a natural membrane-binding protein (or would do so but for the degeneracy of the genetic code), for example under stringent conditions (such as at approximately 35°C to 65°C in a salt solution of approximately 0.9 molar).

In a more broadly expressed range of embodiments of the invention, the particle-forming protein and the membrane-bound protein may be so chosen, or either or both of them may be so modified, that they are packaged efficiently together. This may be particularly useful when the particles are intended for immunogenic purposes as better immunogenicity, increased avidity and/or improved targeting may well result from more efficient packaging.

The membrane-bound protein may confer on the particles enzymic activity and/or cytotoxic activity. Either or both of these activities may be useful in human or veterinary therapy or diagnosis, as may any other pharmaceutical activity. T cell proliferative activity, such as is exhibited by the molecule BBI, is another example.

It will be appreciated that the invention is not restricted to having a single population of membrane-bound proteins in the particles. Indeed it could well be advantageous to have, for example, proteins which confer site-specific targeting ability and proteins which are cytotoxic. Particles having such dual populations could be useful, to give an example, as " magic bullets ": cytotoxic activity could be precisely delivered by this means to tumour cells or cells causing an infection. Another example would be the efficient delivery of a protein, having an effect on a particular group of cells, to those cells; BBI, which has a proliferative effect on T cells, as mentioned above, could be delivered with improved efficiency to T cells by means of a protein such as VCAM having lymphocyte specificity.

Natural membrane-bound proteins will often naturally have one or more of the biological activities described above, or indeed any other biological activity. Such proteins may be chosen for use in particles of the invention for that reason. However, the invention is not limited to their use. Proteins which have the ability to bind to membranes may be modified to have an additional biological activity; and proteins which already have a desired activity may be modified to bind to membranes. Proteins may be prepared which comprise a first amino acid sequence which has membrane-binding activity and a second amino acid sequence which has the desired biological activity, for greatest flexibility. The second amino acid sequence may be identical to at least part of a natural protein, but it does not have to be. Homologues or analogues of natural proteins, or even entirely artificial sequences may be used, as long as the activity is appropriate.

Membrane-bound proteins useful in the invention may alternatively or additionally have any of the properties or characteristics described above in relation to the optional second amino acid sequence of the self-assembling protein molecule described above.

While a number of different characteristics for the components of particles of the invention have been described here, it should be realised that the examples given are illustrative rather than exhaustive. One of the advantages of the invention is the great flexibility it offers in terms of the properties of the particles.

It is not intended that the components of particles of the invention discussed above be necessarily the only components, although many times that will be preferred. Other components can be present as desired, depending on the intended application of the invention.

Particles of the present invention can be prepared by means of recombinant DNA technology in a manner which does not require the presence of replication-competent retroviruses.

According to a second aspect of the invention, there is provided a process for the preparation of a proteinaceous, lipid-containing particle, the process comprising co-expressing in a host cell (a) protein moieties which are capable of self-assembly, wherein the resulting assembly is capable of budding off from the host cell thereby to acquire an at least partial envelope of lipid, and (b) membrane-bound protein sequence.

The membrane-bound protein generally becomes associated with the particles of the invention when the core assembly acquires the lipid on budding off from the host cell. The lipid membrane surrounding the particles has been shown to contain the membrane-bound protein in an active form. The use of recombinant DNA technology to express only the two requisite proteins obviates the need for replication-competent retroviruses for the synthesis of these structures.

Expression will take place in a suitable host cell, which will generally be of animal origin. Mammalian cells may form suitable host cells, as may insect cells, such as those of the genus *Spodoptera*, particularly *Spodoptera frugiperda*. The expression systems will be chosen to be appropriate for the host cell; for insect cells a baculovirus expression system, involving a recombinant baculovirus, is preferred. The baculovirus polyhedrin promoter is a preferred promoter, but other promoters may be used as necessary or desirable. Expression of the self-assembling and membrane-bound proteins may be under differing temporal control to ensure that the maximum efficiency of incorporation of the membrane-bound protein into the lipid-enveloped particles occurs.

The self-assembling protein and the membrane-bound protein may be expressed under the control of the same or different promoters. Generally, each will be expressed under the control of a respective promoter, for example as discussed above, but the use of a common promoter to express, for example, a single fusion protein which is optionally subsequently cleaved to a self-assembling protein and a membrane-bound protein is well within the possibilities contemplated by the invention.

It will be appreciated that, as initially expressed, the self-assembling protein may not instantly be capable of self-assembly; some modification or other event (not least the expression of a second molecule) may be needed before self-assembly takes place. Similarly, some modification or other event may be necessary before the membrane-bound protein actually becomes bound to a membrane, but that affects neither the working of the invention nor the meaning of the term "membrane-bound".

Vectors and host cells useful in the above process themselves form part of the invention.

According to a third aspect of the invention, there is provided an animal cell expression vector comprising first and second coding sequences, wherein the first coding sequence encodes a protein moiety which is capable of self-assembling into an assembly of proteins which is in turn capable of budding off from an animal cell to form a lipid-enveloped particle, and wherein the second coding sequence encodes a membrane-bound protein moiety, the coding sequences being operatively linked, optionally respectively, to a heterologous promoter.

According to a fourth aspect of the invention, there is provided a set of animal cell expression vectors, the set comprising:

(a) a first vector having a coding sequence encoding a protein moiety which is capable of self-assembling into an assembly of proteins which is in turn capable of budding off from an animal cell to form a lipid-enveloped particle, the coding sequence being operatively linked to a heterologous promoter; and

(b) a second vector having a coding sequence encoding a membrane-bound protein moiety, the coding sequence being operatively linked to a heterologous promoter.

According to a fifth aspect of the invention, there is provided a host cell comprising an expression vector or a set of expression vectors as described above.

Particles produced as described above will generally be substantially pure. Purification of the particles may be effected by their chemical and/or physical properties.

Particles of the invention have many uses. Some uses have been described above in relation to the properties of the self-assembling protein or the membrane-bound protein or both. Indeed the uses to which particles of the invention can be put will to a large extent depend on the properties of the proteins chosen for those two roles. Many uses will be therapeutic (whether for humans or animals) or diagnostic. The invention therefore provides, in a sixth aspect, a pharmaceutical or diagnostic composition comprising particles as described above and a suitable carrier.

One primary pharmaceutical use is in the preparation of vaccines, a particular form of pharmaceutical formulation to which the invention extends and which will generally be sterile. Vaccines of the invention may in principle be used in the treatment or prophylaxis of any infectious disease, however mediated (for example of viral, bacterial or protozoal origin). Vaccines may contain one or more appropriate adjuvants and/or other excipients, although it may be found that the components of the particle have a sufficient adjuvant-like effect.

Particles in accordance with the invention may also be used for diagnostic purposes. The particles may be detectably labelled. One of the flexibilities afforded by the invention is that any or all of the components may be labelled, whether with a radionuclide, a spin label or any other detectable label.

As previously indicated other utilities of particles of the invention include: delivery systems for proteins – (such as cytotoxic and/or enzymatic proteins) or nucleic acids (such as ribozymes, antisense RNA, RNAses or DNA or mRNA sequences); fusogenic systems for cytoplasmic delivery; and other systems.

Preferred features of all the aspects of the invention are as described generally above.

The invention will now be illustrated by the following examples with reference to the accompanying drawings in which:

FIGURE I is a schematic diagram of the construction of plasmid pOGSI5;

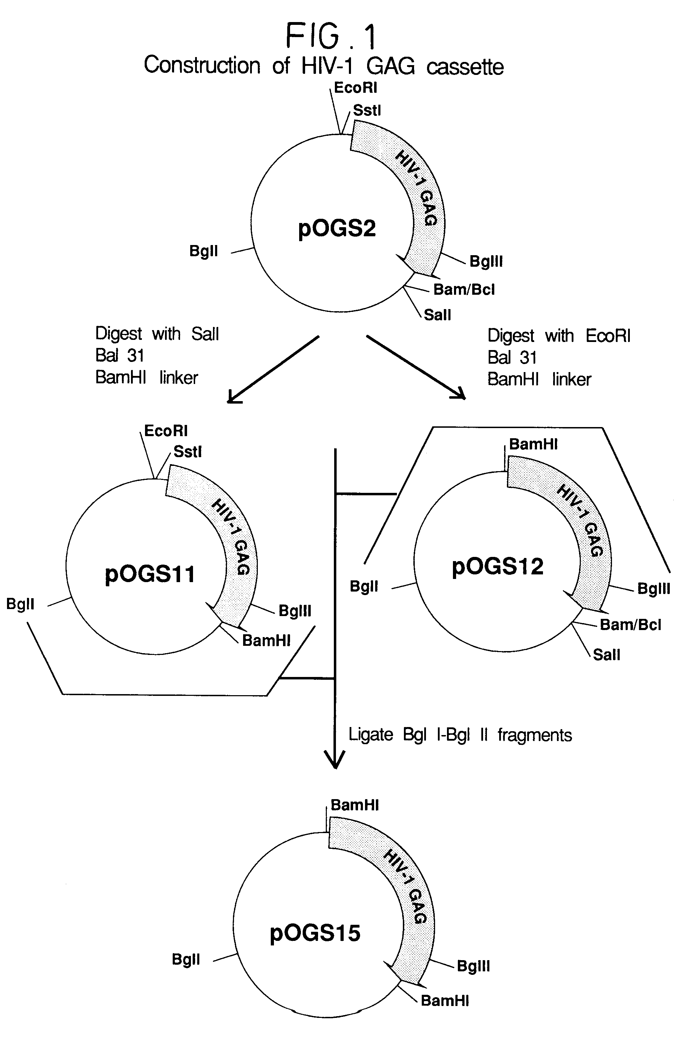

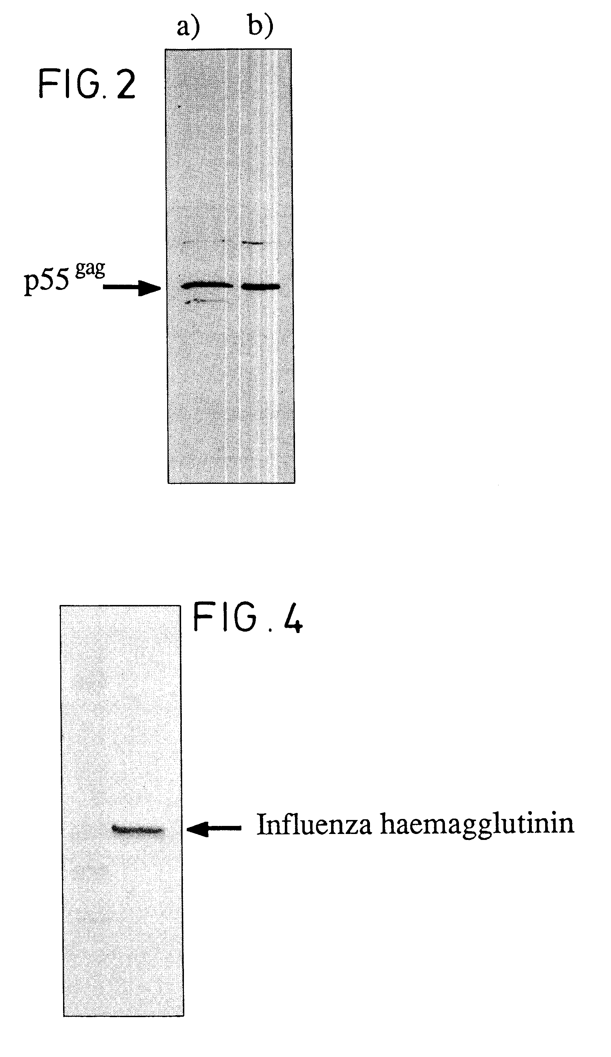

FIGURE 2 is a Western blot, developed using an anti-p24 monoclonal antibody. Track A) is the cell pellet following the infection of *Spodoptera frugiperda* cells with a recombinant baculovirus expressing p55$^{gag}$. Track B) is the culture supernatant of *Spodoptera frugiperda* cells infected with a recombinant baculovirus expressing p55$^{gag}$;

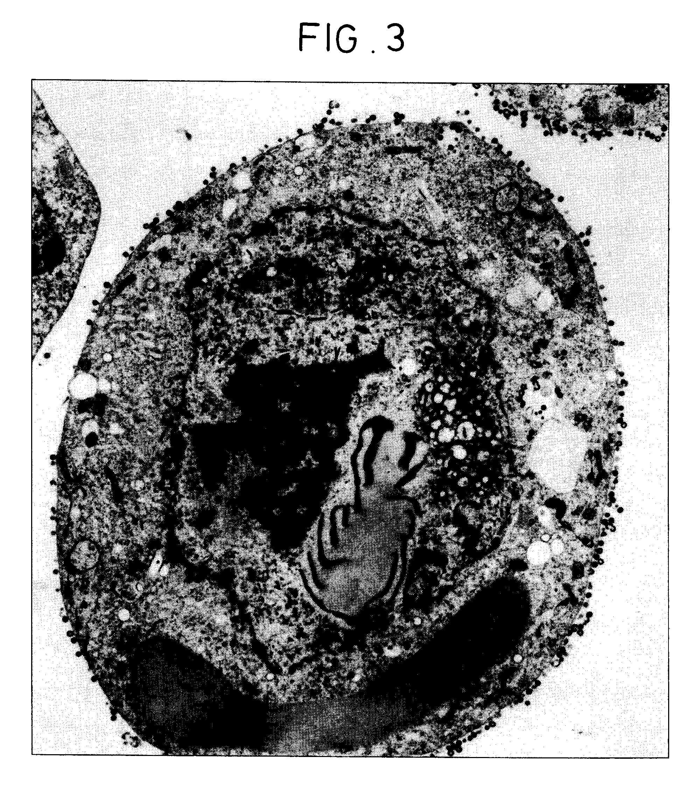

FIGURE 3 is an electron micrograph of a thin section of *Spodoptera frugiperda* cells infected with a recombinant baculovirus expressing p55$^{gag}$;

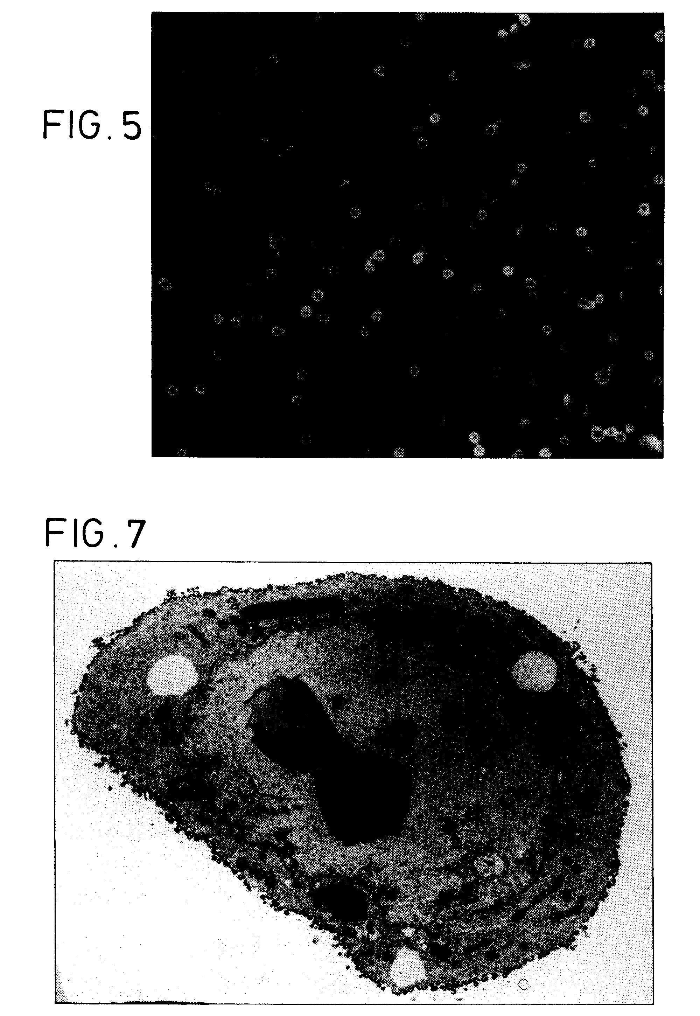

FIGURE 4 shows a Western blot of *Spodoptera frugiperda* cells infected with a recombinant baculovirus expressing influenza haemagglutinin; (the antibody used was R355$\alpha$ A/PR/8 HA obtained from the National Institute of Biological Standards and Control);

FIGURE 5 shows a fluorescence photomicrograph of *Spodoptera frugiperda* cells infected with a recombinant baculovirus expressing influenza haemagglutinin and treated with rabbit anti-HA and FITC-labelled goat anti-rabbit IgG antibodies;

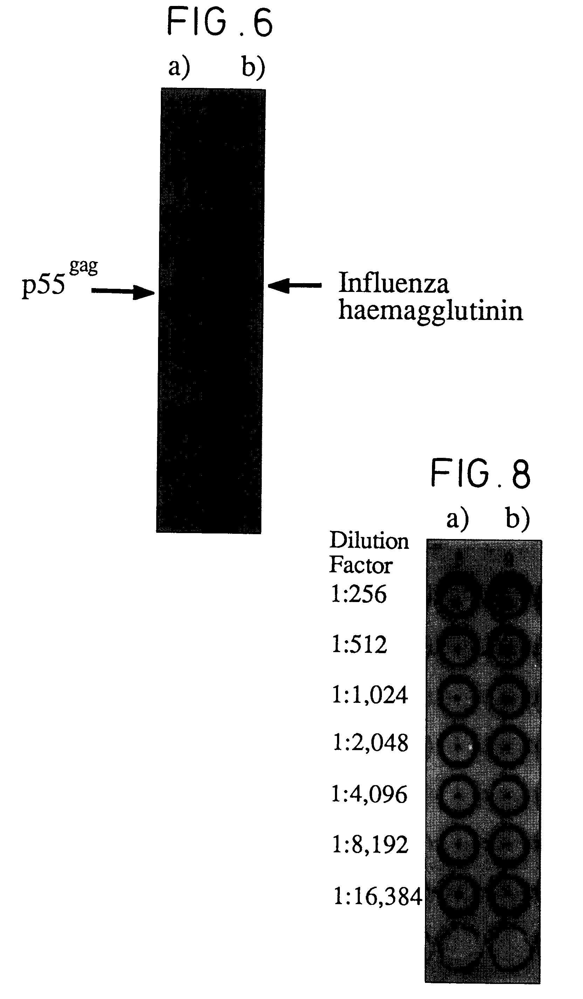

FIGURE 6 shows a Western blot of particles recovered a discontinuous sucrose density gradient of culture supernatant of *Spodoptera frugiperda* cells co-infected with recombinant baculoviruses expressing influenza haemagglutinin and p55$^{gag}$. a) shows the results using an anti-p24 antibody; b) shows the result using an anti-haemagglutinin antibody;

FIGURE 7 is an electron micrograph of a thin section of an *Spodoptera frugiperda* cell co-infected with recombinant baculoviruses expressing p55$^{gag}$ and influenza HA;

FIGURE 8 shows a photograph of a haemagglutination assay performed using particles recovered from a discontinuous sucrose gradient of culture supernatant from a) *Spodoptera frugiperda* cells infected with a recombinant baculovirus expressing p55$^{gag}$; b) *Spodoptera frugiperda* cells co-infected with recombinant baculoviruses expressing p55$^{gag}$ and haemagglutinin;

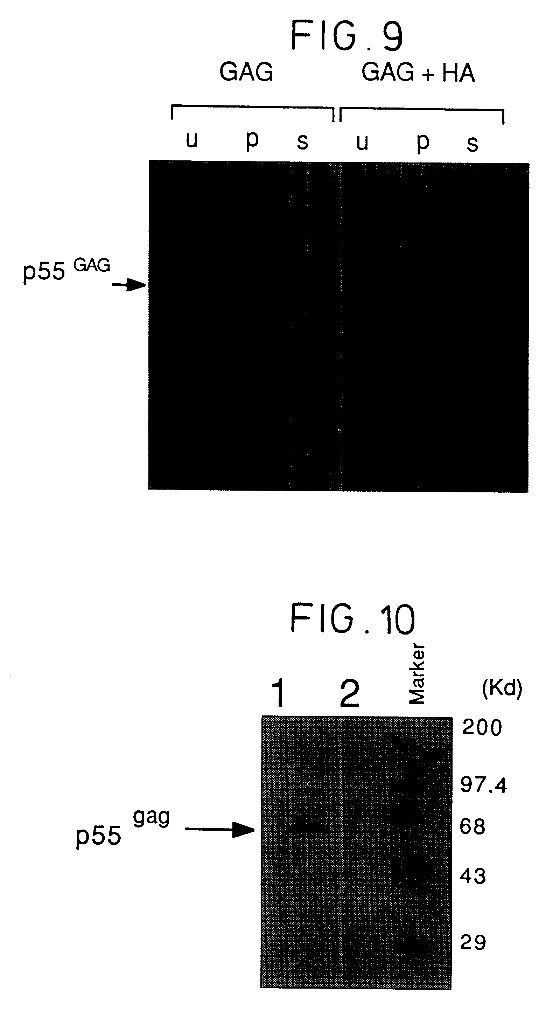

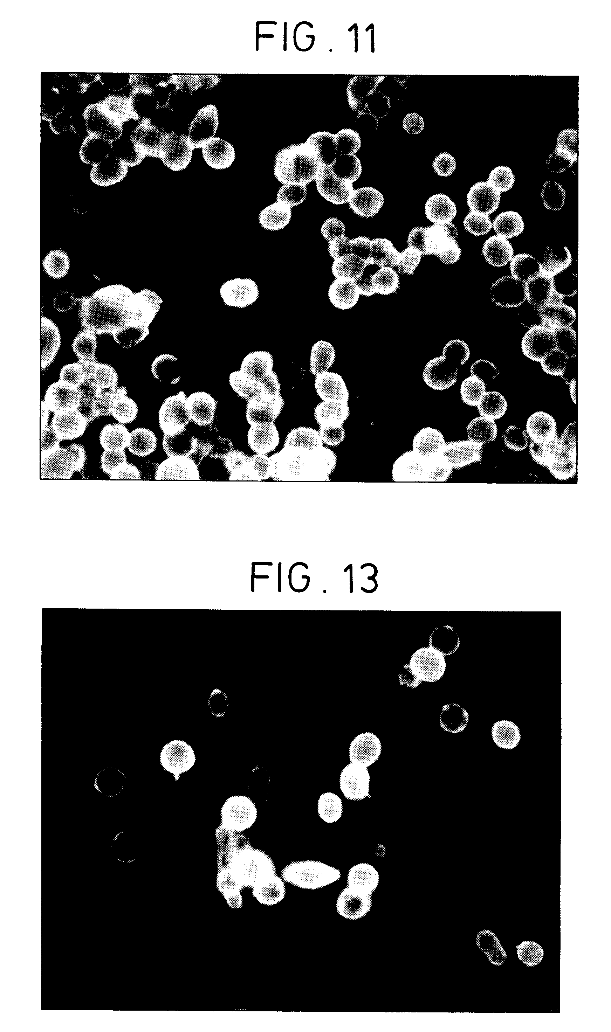

FIGURE 9 shows a Western blot, developed with an anti-p24 monoclonal antibody, of p55$^{gag}$ particles and p55$^{gag}$ particles/haemagglutinin particles adsorbed to chick red cell ghosts. Lanes marked u) correspond to unadsorbed material, lanes marked p) correspond to the red cell ghost pellet and lanes marked s) correspond to the supernatant after adsorption of the particles to red cells;

FIGURE I0 shows a Western blot, developed with an anti-p24 monoclonal antibody, of p55$^{gag}$-haemagglutinin virus-like particles adsorbed to chick red blood cells in the absence (lane I) or presence (lane 2) of an anti-haemagglutination anti-serum (F7/80/$\alpha$A/PR/8);

FIGURE II shows a fluorescence photomicrograph of *Spodoptera frugiperda* insect cells infected with a recombinant baculovirus expressing human ICAM-I (AcOGS722) and treated with mouse anti-ICAM-I monoclonal antibodies and FITC-labelled goat anti-mouse IgG antibodies;

FIGURE I2 shows a summary of the manipulations undertaken to express human VCAM and VCAM/gp4I chimearic molecules;

FIGURE I3 shows a fluorescence photomicrograph of *Spodoptera frugiperda* insect cells infected with a recombinant baculovirus expressing human VCAM (AcOGS738) and treated with mouse anti-VCAM monoclonal antibodies and FITC-labelled goat anti-mouse IgG antibodies;

FIGURE I4 shows a fluorescence photomicrograph of *Spodoptera frugiperda* insect cells infected with a recombinant baculovirus co-expressing human VCAM and HIV-I gag (AcOGS740), and treated with mouse anti-VCAM monoclonal antibodies and FITC-labelled goat anti-mouse IgG antibodies;

FIGURE I5 shows Western blot analysis on cell lysates of AcOGS740- infected insect cells. The blots were developed with either an anti-p24 monoclonal antibody (A) or polyclonal anti-human VCAM anti-serum (B). Co-expression of both HIV-I p55$^{gag}$ and human VCAM is confirmed;

FIGURE I6 shows a photograph of rosettes of U937 cells bound to adherent AcOGS740-infected insect cells at both 37°C (upper panel) or 4°C (lower panel);

FIGURE I7 shows development of syncytia in AcVSV-G-infected insect cells (upper panel) incubated at pH 5.5. AcNPV-infected cells (lower panel), also maintained at pH 5.5, are included for comparison;

FIGURE I8 shows SDS-PAGE and Western blot analyses of p55$^{gag}$ particles isolated from cells co-expressing VSV-G protein. Panel A represents a protein gel stained with Coomassie Brilliant Blue and demonstrates co-purification of p55$^{gag}$ and VSV-G protein. Authenticity of these proteins is provided by Western blot analysis developed with an anti-p24 monoclonal antibody (panel B), or an anti-VSV anti-serum (Stratech Scientific Ltd) (panel C);

FIGURE I9 shows the development of syncytia in insect cells after adsorption of p55$^{gag}$/VSV-G particles and brief exposure to low pH medium to induce the fusogenic activity of VSV-G (upper panel). p55$^{gag}$ particles did not induce syncytia formation in insect cells (lower panel);

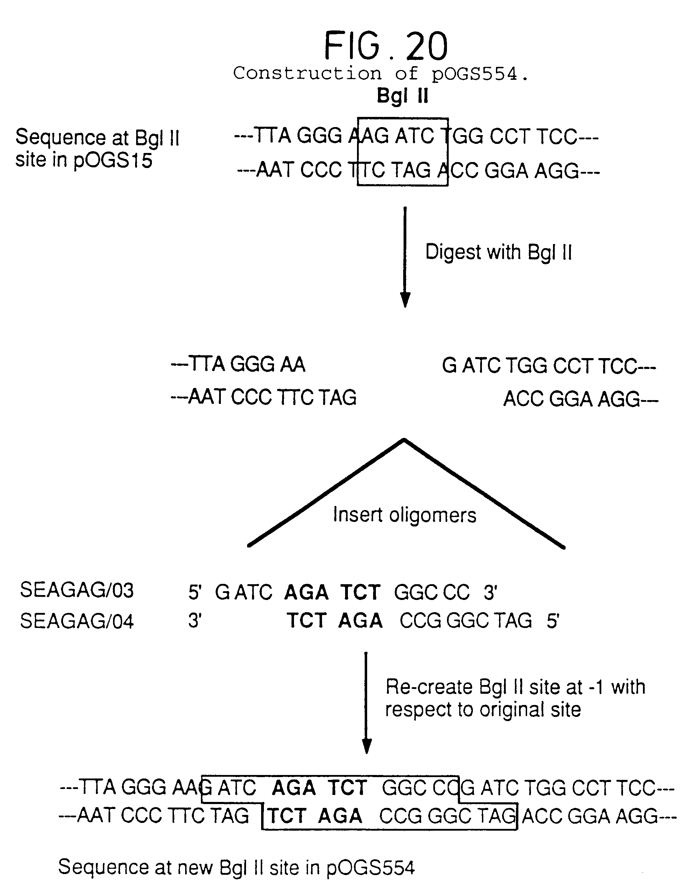

FIGURE 20 shows the sequence details of the construction of pOGS554;

FIGURE 2I is a schematic diagram of the construction of pOGS554, pOGS555, pOGS562 and pOGS563;

FIGURE 22 shows the sequence details of the construction of pOGS562;

FIGURE 23 is a schematic diagram of the general purpose baculovirus expression vector pAcRP23;

FIGURE 24 shows a Western blot of extracts from insect cells infected with wild-type baculovirus AcNPV, AcKIgag (full-length GAG) or AcOGS556 (full-length GAG/GPGR loop). a) shows the result using anti-GAG monoclonal antibody; b) shows the result using anti-GPGR monoclonal antibody;

FIGURE 25 is an electron micrograph of a thin section of an insect cell infected with AcKIgag (full-length GAG);

FIGURE 26 is an electron micrograph of full-length GAG particles budding from an insect cell infected with AcKIgag;

FIGURE 27 is an electron micrograph of full-length GAG particles after budding from an insect cell infected with AcKIgag;

FIGURE 28 is an electron micrograph of a thin section of an insect cell infected with AcOGS556 (full-length GAG/GPGR loop);

FIGURE 29 is an electron micrograph of semi-particulate structures seen in the nucleus of insect cells infected with AcOGS556 (full-length GAG/GPGR loop);

FIGURE 30 shows a Western blot of extracts from insect cells infected with wild-type baculovirus AcNPV, AcOGS572 (truncated GAG) or AcOGS574 (truncated GAG/GPGR loop). a) shows the result using anti-GAG monoclonal antibody; b) shows the result using anti-GPGR monoclonal antibody;

FIGURE 3I is an electron micrograph of a thin section of an insect cell infected with AcOGS572 (truncated GAG);

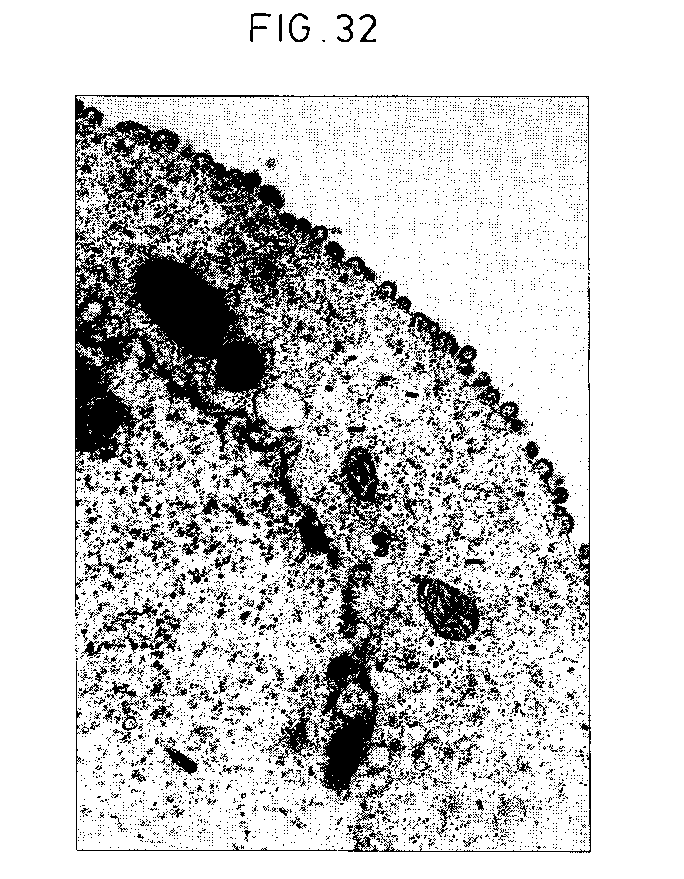

FIGURE 32 is an electron micrograph of truncated GAG particles budding from an insect cell infected with AcOGS572;

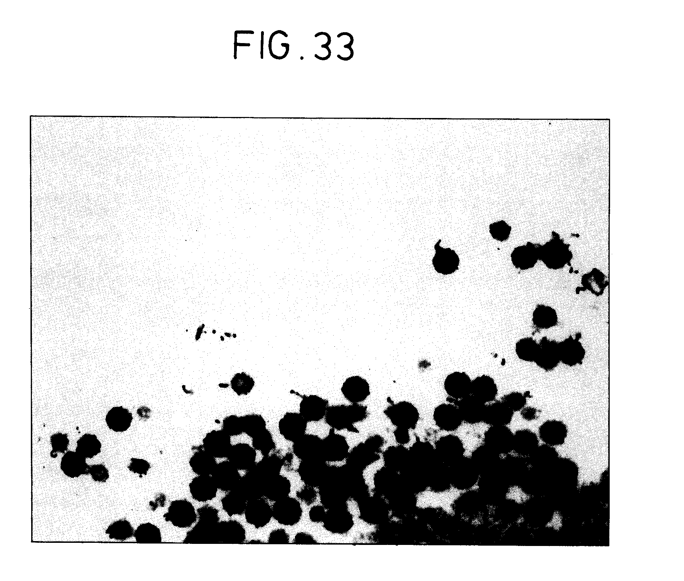

FIGURE 33 is an electron micrograph of truncated GAG particles after budding from an insect cell infected with AcOGS572;

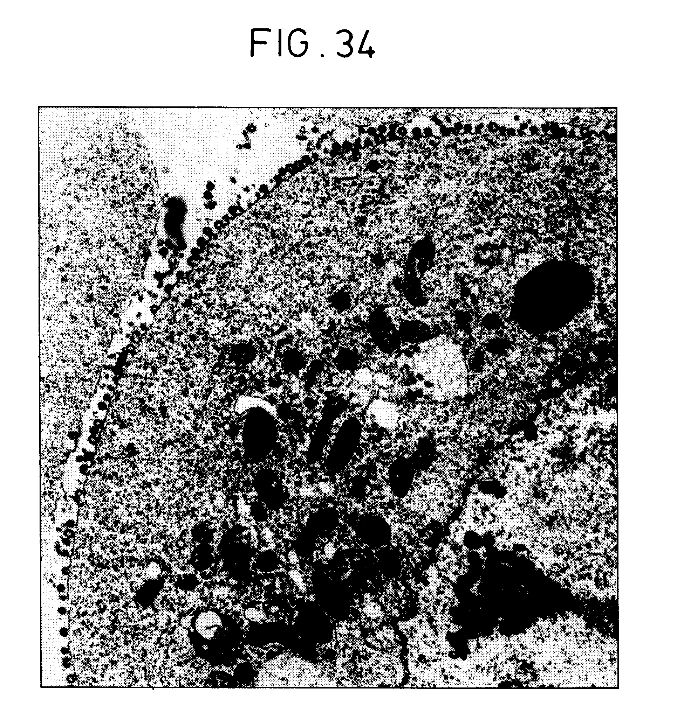

FIGURE 34 is an electron micrograph of a thin section of an insect cell infected with AcOGS574 (truncated GAG/GPGR loop);

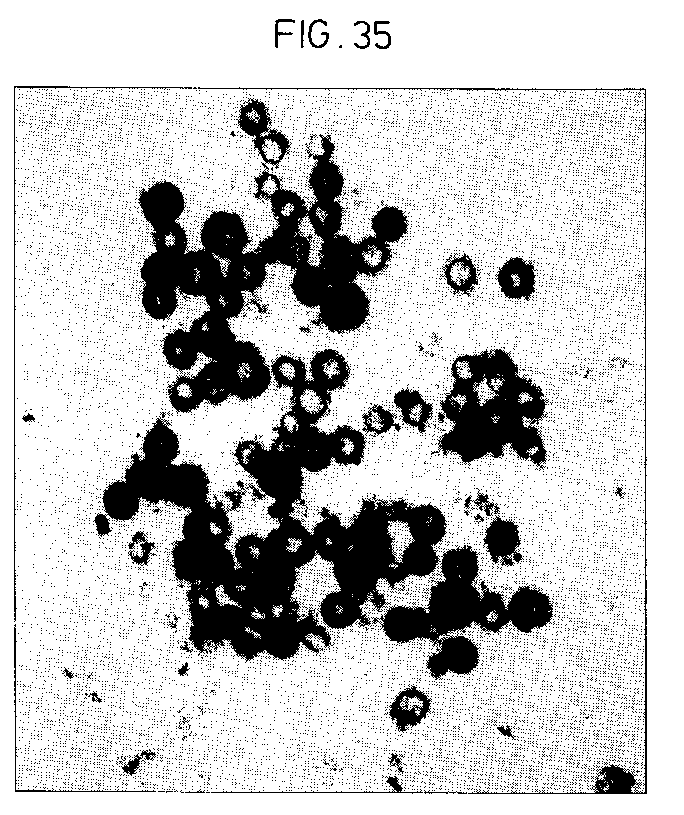

FIGURE 35 is an electron micrograph of hybrid GAG:GPGR loop particles in the nucleus of insect cells infected with AcOGS574; and

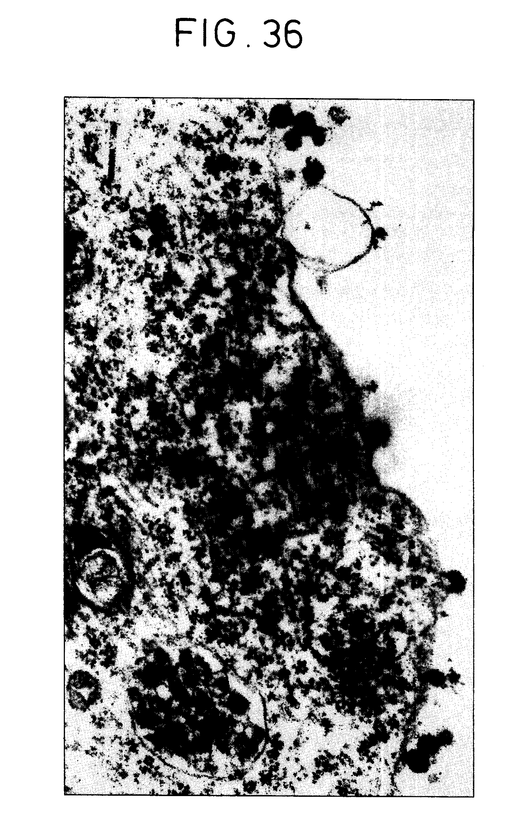

FIGURE 36 is an electron micrograph of hybrid GAG:GPGR loop particles budding from insect cells infected with AcOGS574.

EXAMPLE I

The *E. coli* strain used was HW87 (*ara*DI39, (*ara-leu*)del 7697, (lacIPOZY)delI74, *gal*U, *ga*K, *hsd*R, *rps*L, *srl*, *rec*A56). The use of this strain is not essential; those skilled in the art will appreciate that other strains may readily be substituted. *E. coli* media were prepared according to Miller (Miller, 1972, "Experiments in Molecularar Genetics", CSH p433). *E. coli* were transformed using standard methods (Maniatis et al, 1982, "Molecular Cloning - A Laboratory Manual", CSH p199).

Standard procedures were used for restriction digestion and plasmid constructions (Maniatis *et al*, 1982 *op. cit*). Restriction enzymes and T4 DNA ligase were used according to the suppliers'instructions.

Plasmid DNA was isolated from *E. coli* preparatively as described by Chinault and Carbon (Chinault and Carbon, 1979 *Gene* **5**, 111) and for rapid analysis by the method of Holmes and Quigley (Holmes and Quigley, 1981, *Anal. Biochem.* **114**, 193).

HIV-I gag constructs were derived from pOGS2. Plasmid pOGS2 contains nucleotides 678-2470 of HIV-I (isolate BH 10) inserted into plasmid pSP64 (Promega Corp) as an *Ssf*I-*Bcl*I restriction fragment.

The gag gene is encoded by nucleotides 790-2328. In order to provide the *gag* gene on a convenient BamHI fragment, pOGS2 was modified using *Bal*3I exonuclease and *Bam*HI linkers, as follows: to modify the 3' end of the gag fragment, plasmid OGS2 was cleaved with *Sal*I, digested with *Bal*3I exonuclease for various times and re-ligated in the presence of excess *Bam*HI linkers (CCGGATCCGG). The deletion end points of the resulting plasmids were determined by DNA sequencing. Plasmid pOGS 11 is a deletion derivative in which the BamHI site is at position +49 with respect to the gag termination codon. To modify the 5' end of the gag fragment, pOGS2 was cleaved with *Eco*RI, digested with *Bal*3I exonuclease for various times and re-ligated in the presence of excess *Bam*HI linkers (CCGGATCCGG). The deletion end points were determined by DNA sequencing. Plasmid pOGS 12 is a deletion derivative in which the *Bam*HI site is at position -22 with respect to the gag initiating ATG codon. In order to provide a *gag* gene flanked by *Bam*HI sites, the 3'end of the *gag* gene in pOGSI2 was replaced with a *Bgl*II-*Bgl*I fragment from pOGSII containing the modified sequence. The resulting plasmid was designated pOGSI5 (see Figure I).

The entire *gag* coding sequence was excised from pOGSI5 as a *Bam*HI cassette and cloned into the baculovirus expression vector pAcR23 to generate pAcKIgag. The vector pAcRP23 was constructed at The Institute of Virology and Environmental Microbiology, Oxford, UK, by cloning a restriction fragment (*Eco*RI-I) encompassing the polyhedrin gene of *Autographa californica* nuclear polyhedrosis virus into a high copy number bacterial plasmid (pUC8). Part of the polyhedrin gene was deleted and replaced by a unique restriction enzyme site (*Bam*HI) for insertion of foreign genes (see Matsuura *et al*, 1987, *J. Gen. Virol.* **68**, 1233-1250 for details of baculovirus expression vector construction). When cloned into this vector, the *gag* gene comes under control of the very powerful polyhedrin promoter.

The cell line designated *Spodoptera frugiperda* 9 (Sf9) (Summers, M. D. & Smith, G. E. 1987. *Texas Agricultural Experiment Station Bulletin* No. 1555) which is publicly available and is a clonal derivative of the lepidopteran line IPLB-Sf21-AE Vaughn, J. L. *et al*, 1977. *In Vitro* **13**, 213-217) was used for all experiments. This was grown in suspension culture in medium comprising TC100 (GIBCO-BRL, UK) containing 10% heat-inactivated foetal calf serum (ICN-FLOW Laboratories, UK) plus 50IU/ml penicillin and 50mg/ml streptomycin (GIBCO-BRL, UK).

Recombinant baculoviruses were prepared by using the phenomenon of in vivo recombination between homologous DNA sequences. The viral (AcNPV) DNA and plasmid DNA (pAcKIgag) were introduced into *Spodoptera frugiperda* insect cells by the method of calcium chloride precipitation and progeny recovered after 48h (Summers, M. D. & Smith, G.E. 1987. *Texas Agricultural Experiment Station Bulletin* No. 1555). A recombinant baculovirus (designated AcKI-GAG) was selected on the basis of its polyhedrin-negative phenotype according to the procedure of Brown and Faulkner (Brown, M. & Faulkner, P., 1977, J. *Gen. Virol.* **36**, 361-364). After plaque purification, high titre viral stock of the recombinant baculovirus-encoding the expression of HIV-I *gag*, were prepared and their titre determined in a plaque assay (titres were in excess of $5x10^7$ plaque forming units (pfu) per ml).

When *Spodoptera frugiperda* insect cells were infected with AcKIgag-recombinant baculovirus, the p55$^{gag}$ polypeptide is expressed in infected cells and can be detected by Western blotting using an anti-p24 monoclonal antibody (see Figure 2). It is apparent that p55$^{gag}$ is transported to the plasma membrane where it is released from the cell, since p55$^{gag}$ accumulates in the culture supernatant (Figure 2). Electron microscopy of AcKI-GAG infected cells shows that the release of p55$^{gag}$ is due to the formation of particles which bud from the cell membrane (Figure 3) and also Gheysen, *et al*, 1989, *Cell* **59**, 103-112).

EXAMPLE 2

A recombinant baculovirus expressing the haemagglutinin gene from influenza virus (Strain A/PR/8/34) was that previously used by Possee (Possee, 1986, *Virus Research* **5**, 43-59). High titre viral stock of this virus (designated AcRPI8.HA) were also prepared. Western blotting, using an anti-haemagglutinin serum provided by the National Institute of Biological Standards and Control, confirms the expression of haemagglutinin in *Spo-*

*doptera frugiperda* cells infected with the recombinant baculovirus AcRPI8.HA (Figure 4). Immunofluorescence of *Spodoptera frugiperda* cells infected with AcRPI8.HA using anti-HA (ex-NIBSC) at a dilution of I:50 and FITC-labelled goat anti-rabbit IgG (Sigma, Poole, UK) gave a staining pattern consistent with the HA being located in the plasma membrane (Figure 5). This demonstrates that the recombinant HA produced in the AcRI8.HA-infected cells is processed and transported as in a natural infection.

In order for budding p55$^{gag}$ particles to acquire haemagglutinin on their surface it is necessary to express of p55$^{gag}$ and haemagglutinin in the same cells. This was achieved in the following way. *Spodoptera frugiperda* insect cells were maintained in log growth and grown to a cell density of approximately I. 5xl0$^6$/ml before use. Prior to infection, cells were spun down at 900rpm, 20°C, for 8 min, and the culture supernatant discarded. Cells were infected with a mixture of both AcKI-GAG and AcRPI8.HA such that an moi (multiplicity of infection) of 2pfu for each virus per cell was achieved. This ensured that a co-infection was established in all cells with the consequent expression of both foreign genes.

Alternatively, to express p55$^{gag}$ only, cells were then infected with AcKI-GAG at an moi of 2 plaque forming units (pfu) per cell. After Ih at room temperature, the infected cells were pelleted as before, and the viral inoculum removed. Cells were re-suspended in fresh medium to a density of I.5xl0$^6$ cells/ml and incubated as a suspension culture at 27°C for 66h.

The culture was decanted into sterile 50ml centrifuge tubes and spun at 3krpm, 4°C, for I0 min to pellet the cells. Cleared supernatant was loaded onto sucrose step gradients (60 % sucrose overlayered with 30 % sucrose both in TEN: I0mM Tris-HCl pH 7.4, ImM EDTA, I50mM NaCl) and centrifuged at 96,000g, I0°C for 2h. Under these centrifugal forces monomeric influenza haemagglutinin does not sediment through the 30% sucrose. By contrast, haemagglutinin associated with particles will have a greatly increased apparent sedimentation coefficient and therefore will sediment through the 30% sucrose. Particles were recovered from the 30%/60% sucrose interface, and after pooling of the material from several gradients, further concentrated on a similar sucrose step gradient. Following recovery, the samples were dialysed overnight against TEN at 4°C to remove the sucrose. Western blotting of particles recovered from the 30 %/60 % sucrose interface showed the presence of both haemagglutinin and p55$^{gag}$ (Figure 6). This confirms the particulate nature of p55$^{gag}$ and influenza haemagglutinin. Electron microscopy of thin sections of co-infected cells (Figure 7) shows the presence of budding particles indistinguishable in morphology from those produced from cells infected with AcKI-GAG alone.

## EXAMPLE 3

The activity of the haemagglutinin containing particles recovered from the 30% /60% interface in Example 2 above was assayed using a standard haemagglutination assay. One day old chick red blood cells (supplied asl0 % in alseviers; Serotec, UK) were pelleted by centrifugation at 250g, 4°, for 5 min, and washed three times in phosphate buffered saline (PBS) and their concentration adjusted to I.0% (v/v) in PBS. Serial two-fold dilutions of semi-purified particles were prepared in PBS across a microtitre plate and an equal volume of I% red blood cells added. The 50% end-point was estimated after 60 min at room temperature and the HA titre calculated as the reciprocal of the end point per ml. As can be seen from Figure 8, p55$^{gag}$ when expressed alone and purified as described above, has no haemagglutination activity, whereas when it is coexpressed with HA the haemagglutination titre is I/I28. This confirms that the haemagglutinin associated with the particles is active.

## EXAMPLE 4

A physical association between the p55$^{gag}$ particles and haemagglutinin of Example 2 was demonstrated by haemagglutinin mediated adsorption of p55$^{gag}$ onto chick red blood cell ghosts. Chick red blood cell ghosts were prepared by hypotonic shock (Staufenbiel and Lazarides, I986, *PNAS*, 83, 3I8-322). The cells supplied in alseviers were washed three times in PBS as described above, and after the final centrifugation, the cell pellet was re-suspended in I00 volumes cold (4°C) lysis buffer (5mM Tris-HCl pH 7.6, 5mM MgCl$_2$, ImM EDTA). After a 3 min incubation at 4°C, the ghosts were sedimented at 300g, 4°C for 5 min and washed in a further I00 volumes of lysis buffer. Ghosts were finally re-suspended to a concentration of 5% in TEN containing 0.I% BSA. I00μl aliquots were then centrifuged as described and the supernatants discarded to leave ghost pellets with which the samples could be mixed. 2μl of 5% BSA was added to I00μl sample (p55gag virus-like particles or p55gag virus-like particles presenting influenza haemagglutinin) in an EPPENDORF tube and spun in an EPPENDORF centrifuge (6,000g) for 2 min. (The word EPPENDORF is a trade mark.) I00μl of this cleared sample was added to an aliquot of the pelleted ghosts and incubated at 20°C for I h. The mixture was then spun at 300g, 20°C for 5 min and the supernatant (I00μl) was withdrawn and mixed with I00μl 2X SDS-PAGE loading buffer. The pellet was washed with TEN + 0.I% BSA, then TEN alone and finally lysed in 200μl SDS-PAGE

loading buffer.

Samples were resolved by I0% SDS-PAGE, and blotted onto nitrocellulose filter. The presence of gag protein was detected using a monoclonal antibody to HIV-I p24 (DuPont, USA) in a standard Western blot analysis. As can be seen from Figure 9, the presence of p55$^{gag}$ is only detected in the pellet if the particles are purified from cells co-expressing HA. This demonstrates that the particles formed by budding p55$^{gag}$ are presenting HA, in an active form, in the lipid bilayer surrounding the p55$^{gag}$ particles.

To confirm that binding of the p55$^{gag}$ virus-like particles purified from cells co-expressing influenza haemagglutinin to the chick red blood cell ghosts was mediated by haemagglutinin, the experiment was repeated in the presence of F7/80/αA/PR/8, an antiserum which inhibits haemagglutination (provided by the National Institute of Biological Standards and Control (UK)). This antibody totally prevented binding to the red blood cell ghosts (see Fig I0), thus verifying the interaction to be due to haemagglutinin incorporated into the envelope of the p55$^{gag}$ particles.

## EXAMPLE 5

The human ICAM-I gene, supplied in the vector pCDM8, was obtained from BB Products Ltd, Abingdon, UK. This was manipulated using the standard procedures described in *Example I* to optimize expression in insect cells. These manipulations are detailed below.

The ICAM-I gene was isolated from pCDM8 following digestion with *EAr*I and *Xba*I, and after Klenow repair, ligated into the *Sma*I site of the baculovirus mutagenesis vector pAcCL29. I (Livingstone & Jones, I989, *Nuleic Acid Research* I7:2366) to generate pOGS7I6. This placed the *Bam*HI restriction site of the vector polylinker downstream of the ICAM-I gene. The Kunkel method of site-directed mutagenesis (Kunkel, I985, *Proc. Natl. Acad. Sci. USA*, **82**:488-492) was employed to introduce a *Bam*HI restriction site immediately upstream of the translation initiation codon of ICAM-I to generate pOGS7I8. The ICAM-I gene, now present as a *Bam*HI cassette, was cloned into the baculovirus expression vector pAcYMI (Matsuura *et al*, I987, *J. Gen. Virol.* **68**:I233-I250) to generate pOGS722).

A recombinant baculovirus expressing human ICAM-I was prepared utilising the phenomenon of in vivo recombination between homologous DNA sequences. Viral DNA (AcRP23.LacZ, derived from the Institute of Virology and Environmental Microbiology, Oxford, UK) and plasmid DNA (pOGS722) were introduced into *Spodoptera frugiperda* insect cells by Lipofectin reagent (BRL, UK). Progeny virions, isolated by a Brown and Faulkner plaque assay, were then used to infect *Spodoptera frugiperda* insect cells adhered to multi-well plates. These cells were screened for surface ICAM-I expression by live cell immunofluorescence with a mixture of two monoclonal antibodies raised against human ICAM-I (IIC8I and I4CII, derived from BB Products Ltd, Abingdon, UK) and FITC-labelled goat anti-mouse IgG (Sigma Poole, UK). A recombinant baculovirus (designated AcOGS722) selected in this manner was plaque purified, and high titre viral stock of the recombinant baculovirus-encoding the expression of human ICAM-I were prepared. Virus titres in excess of 5 x I0$^7$ plaque forming units (pfu) per ml were produced (determined by plaque assay).

When *Spodoptera frugiperda* insect cells were infected with AcOGS722, intense surface staining was observed in live cell immunofluorescence with anti-human ICAM-I monoclonal antibodies and FITC-labelled goat anti-mouse IgG (see Figure II). This demonstrates that the recombinant human ICAM-I produced in the AcOGS722-infected cells is processed and transported to the cell surface as in ICAM-I-positive human cells.

As exemplified in Examples 2 to 4, the simultaneous expression of human ICAM-I and p55$^{gag}$ in *Spodoptera frugiperda* insect cells by co-infection with AcOGS722 and AcKI-GAG will result in the incorporation of human ICAM-I into the lipid envelope of the p55$^{gag}$ virus-like particles as they bud from the cell. Consequently these particles will have a tropism for LFA-I bearing cells. Such cells include antigen presenting cells.

## EXAMPLE 6

The human VCAM gene, supplied in the vector pCDM8, was obtained from BB Products Ltd, Abingdon, UK. This was manipulated using the standard procedures described in *Example I* to optimize expression in insect cells. these manipulations are summarized in Figure I2, and are detailed below. The VCAM gene was excised from pCDM8 following digestion with *Xho*I and *Pvu*II. It was then cloned into the vector pUCI8 which had been digested with *Bam*HI, klenow repaired then digested with *Sal*I. This generated the construct pOGS723, and placed the polylinker *Pst*I and *Bam*HI restriction sites upstream and downstream respectively of the VCAM gene. The VCAM gene was then released as a *Pst*I to *Bam*HI fragment and cloned into the baculovirus mutagenesis vector pAcCL29.8 (Livingstone & Jones, I989, *Nucleic Acid Research* I7:2366) also digested with *Pst*I and *Bam*HI to generate pOGS73I. Site-directed mutagenesis was employed to introduce a *Bam*HI restriction site immediately upstream of the translation initiation codon of VCAM to give pOGS734. The VCAM gene, now present as a *Bam*HI cassette, was cloned into the baculovirus expression vector pAcYMI (Matsuura *et al*, 1987, *J. Gen.Virol*. **68**:1233-1250) to generate pOGS738.

A recombinant baculovirus expressing human VCAM was prepared utilizing the phenomenon of in vivo recombination between homologous DNA sequences. Viral DNA (AcRP23.LacZ) and plasmid DNA (pOGS738) were introduced into *Spodoptera frugiperda* insect cells by Lipotectin reagent (BRL, UK). Progeny virions, isolated by a Brown and Faulkner plaque assay, were then used to infect insect cells adhered to multi-well plates. These cells were screened for surface VCAM expression by live cell immunofluorescence with a mixture of two monoclonal antibodies raised against human VCAM (BB Products Ltd, Abingdon, UK) and FITC-labelled goat anti-mouse IgG (Sigma, Poole, UK). A recombinant baculovirus (designated AcOGS738) selected in this manner was plaque purified, and high titre viral stock of the recombinant baculovirus-encoding the expression of human VCAM were prepared. Virus titres in excess of $5 \times 10^7$ plaque forming units (pfu) per ml were produced (determined by plaque assay).

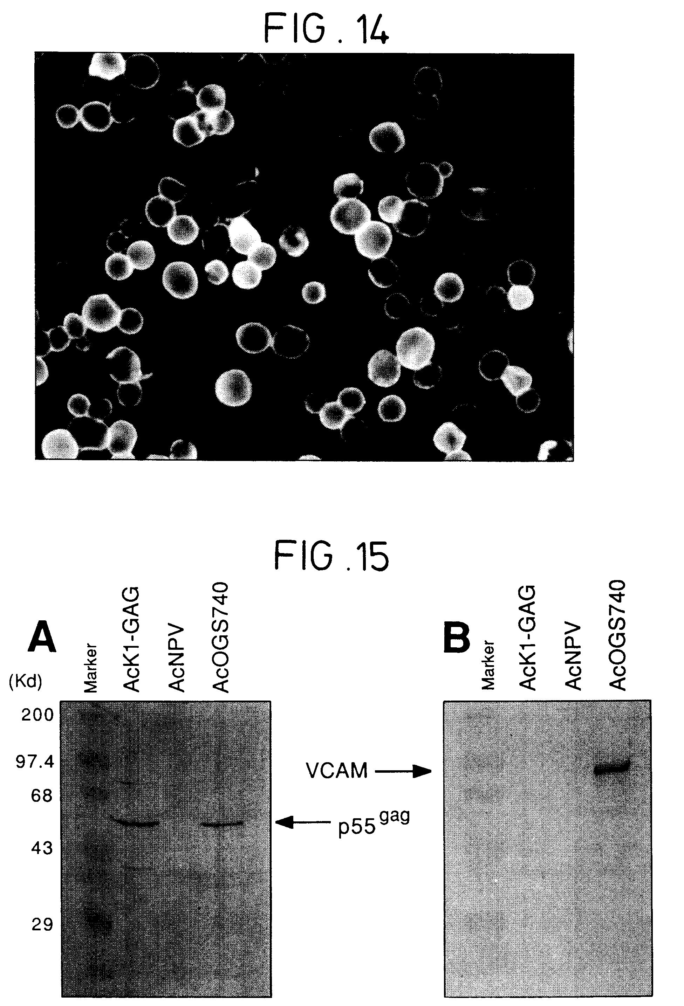

When *Spodoptera frugiperda* insect cells were infected with AcOGS738, intense surface staining was observed in live cell immunofluorescence with anti-human VCAM monoclonal antibodies and FITC-labelled goat anti-mouse IgG (see Figure 13). This demonstrates that the recombinant human VCAM produced in the AcOGS738-infected cells is processed and transported to the cell surface as in VCAM-positive human cells.

Simultaneous expression of human VCAM and p55$^{gag}$ in *Spodoptera frugiperda* insect cells by co-infection with AcOGS738 and AcKI-GAG as exemplified in *Examples 2-4* will thus result in the incorporation of biologically active human VCAM into the lipid envelope of the p55$^{gag}$ virus-like particles as they bud from the cell.

## EXAMPLE 7

Co-expression of two foreign proteins in insect cells can, as described, be successfully achieved by co-infection of *Spodoptera frugiperda* insect cells with two recombinant baculoviruses, each at a high multiplicity of infection. However, the random nature of baculovirus infection means that a proportion of cells will be infected with one or less viruses even at high multiplicities of infections. Therefore, a more efficient approach is to construct a dual recombinant baculovirus containing the two foreign genes, each under control of its own promoter. Infection of insect cells with this dual recombinant will thus result in the simultaneous expression of both foreign proteins in every cell which is infected.

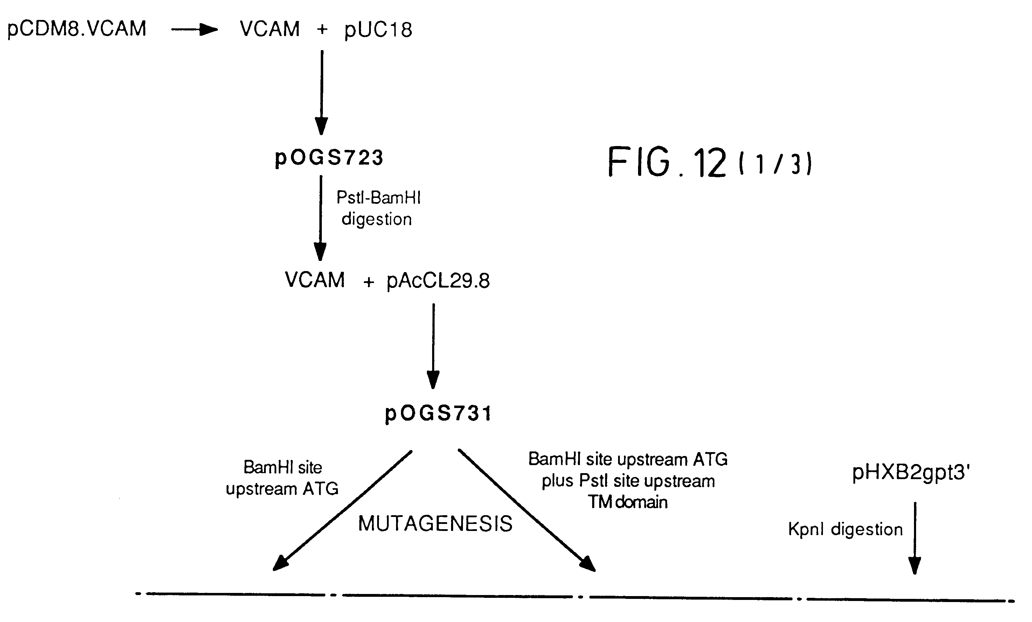

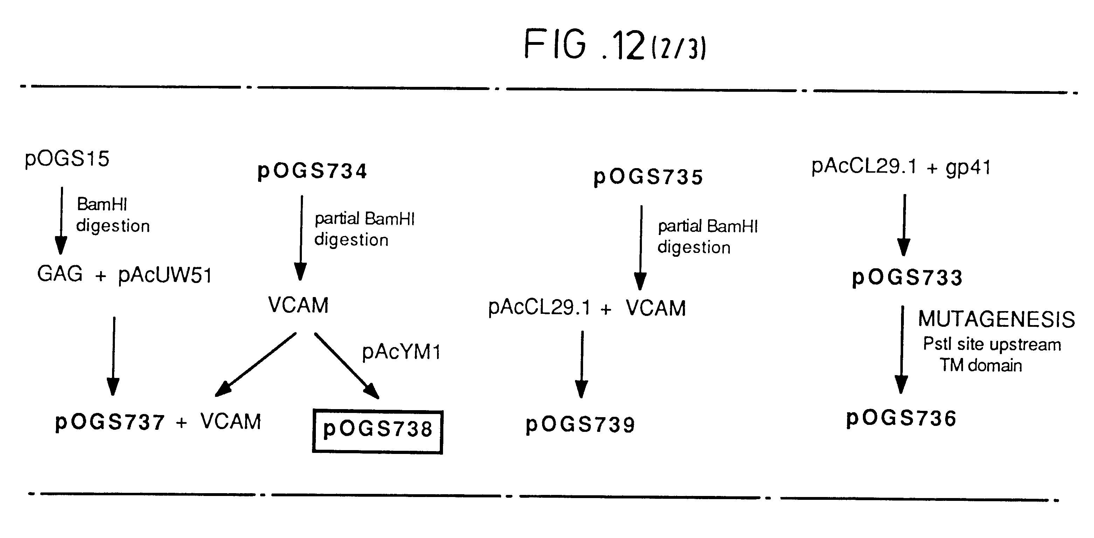

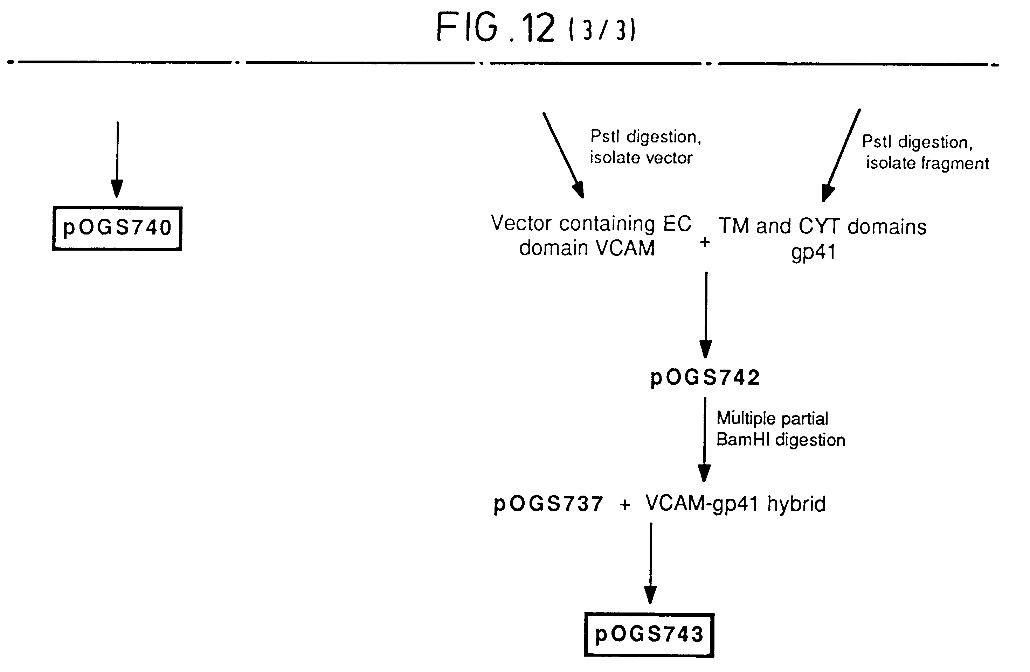

The construction of a dual recombinant baculovirus to co-express HIV-I p55$^{gag}$ and human VCAM is described below, and also summarised in Figure 12.

The entire HIV-I *gag* coding sequence was excised from pOGS 15 as a *Bam*HI cassette and cloned into the *Bgl*II restriction site of the dual baculovirus expression vector pAcUW5I to generate pOGS737. The vector pAcUW5I (derived from pAcUW3, Weyer et al, 1991, J Gen. Virol. **72**:2967-2972) was constructed at the Institute of Virology and Environmental Microbiology, Oxford, UK and contains a polyhedron and a pl0 promoter, both of which are activated very late in the baculovirus infection cycle. Downstream of each of these promoters in the vector is a unique restriction site (*Bam*HI and *Bgl*II respectively) for the insertion of foreign genes. The VCAM gene was excised from pOGS734 as a *Bam*HI fragment, and cloned into the *Bam*HI restriction site of pOGS737 to generate pOGS740. This plasmid thus contains both the HIV-I *gag* and human VCAM genes, and enables a dual recombinant baculovirus to be constructed which will co-express these two proteins simultaneously.

A dual recombinant baculovirus expressing both HIV-I *gag* and human VCAM was prepared by the usual method of in vivo recombination between homologous DNA sequences. Viral DNA (AcRP23.LacZ) and plasmid DNA (pOGS740) were introduced into *Spodoptera frugiperda* insect cells by Lipofectin reagent (BRL, UK). Progeny virions, isolated by a Brown and Faulkner plaque assay, were then used to infect *Spodoptera frugiperda* insect cells adhered to multi-well plates. These cells were screened for surface VCAM expression by live cell immunofluorescence with monoclonal antibodies raised against human VCAM (derived from BB Products Ltd, Abingdon, UK) and FITC-labelled goat anti-mouse IgG (Sigma, Poole, UK). A recombinant baculovirus (designated AcOGS740) selected in this manner was plaque purified, and high titre viral stocks of the recombinant baculovirus-encoding the co-expression of human VCAM and p55$^{gag}$ were prepared. Virus titres in excess of $5 \times 10^7$ plaque forming units (pfu) per ml were produced (determined by plaque assay).

When *Spodoptera frugiperda* insect cells were infected with AcOGS740, intense surface staining was observed in live cell immunofluorescence with anti-human VCAM monoclonal antibodies and FITC-labelled goat anti-mouse IgG (see Figure 14). This demonstrates that the recombinant human VCAM produced in the AcOGS740-infected cells is processed and transported to the cell surface as in VCAM-positive human cells.

Cell lysates of *Spodoptera frugiperda* insect cells infected with AcOGS740 were examined by Western blotting with both an anti-p24 monoclonal antibody (DuPont, USA) and a polyclonal anti-human VCAM antibody (BB Products Ltd). As shown in Figure 15, both p55$^{gag}$ and human VCAM are present in this cell lysate confirming the co-expression of these proteins in AcOGS740-infected cells.

The ligand for VCAM is VLA-4. To confirm that the baculovirus-expressed human VCAM was biologically active, adhesion assays between VCAM-expressing insect cells and human VLA-4 positive cells were performed. The cell line employed, U937, is of a monocytic lineage and was originally derived from a patient suffering from histiocytic lymphoma (Sundstrom & Nilsson, 1976, *Int. J. Cancer*, 17, 565-577). The U937 cells, propagated in RPMI 1640 medium supplemented with 2mM glutamine, 50 U/ml penicillin/streptomycin and 5% heat inactivated fetal calf serum, were harvested, washed in adhesion assay buffer (RPMI 1640 medium containing 50U/ml of penicillin/streptomycin, 2mM glutamine, 10mM HEPES pH7.2, 0.5% BSA), and re-suspended to a cell density of 1 x 10⁷ cells/ml. Monolayers of *Spodoptera frugiperda* insect cells infected with AcOGS740 or wild-type AcNPV were washed with adhesion assay buffer, and 500µl of the U937 cell suspension added. The mixture was then incubated at 37°C for 90 min with regular re-distribution of the U937 cells. The excess U937 cells were then removed, and the insect cells washed twice with adhesion assay buffer and once with PBS before fixing with 0.5 % glutaraldehyde in PBS. The assay was also performed at 4°C with pre-cooled cells in the presence of 0.5 % azide.

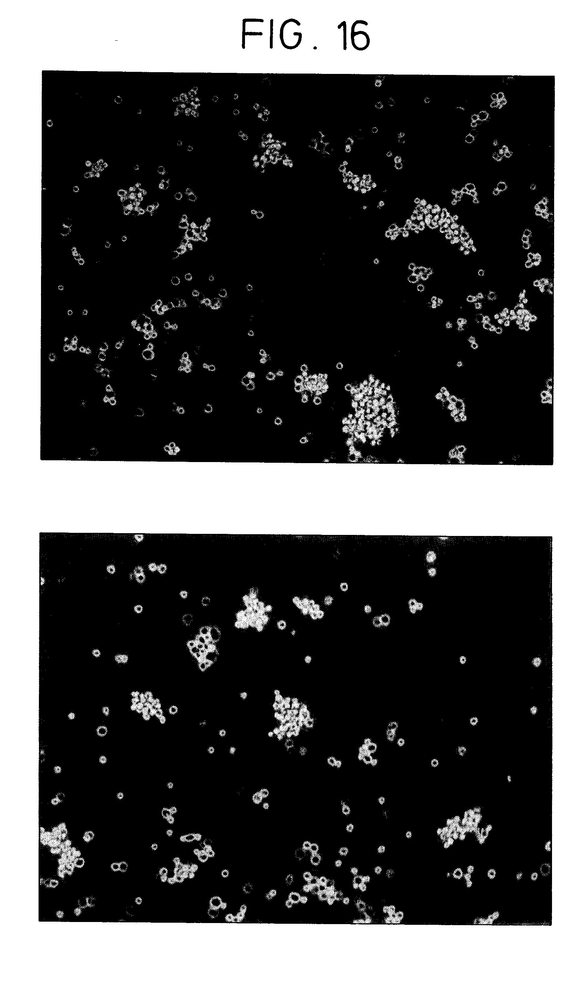

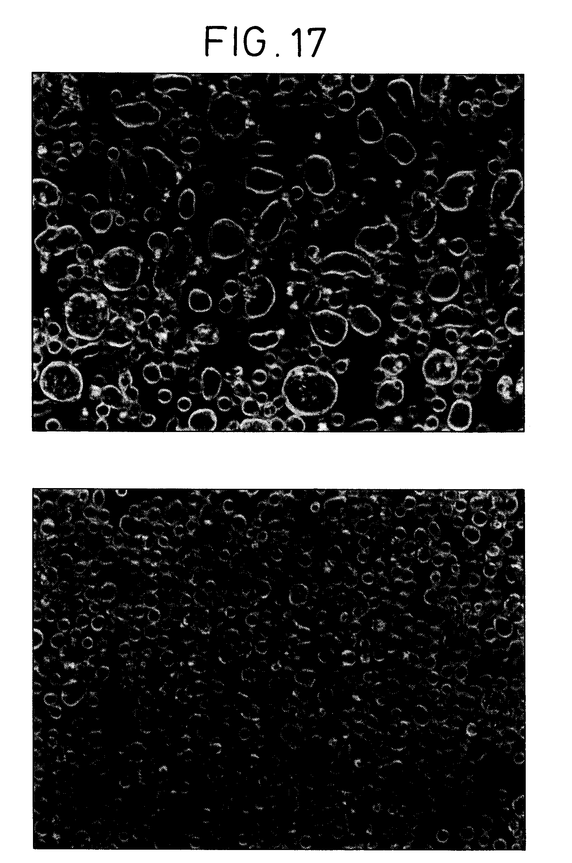

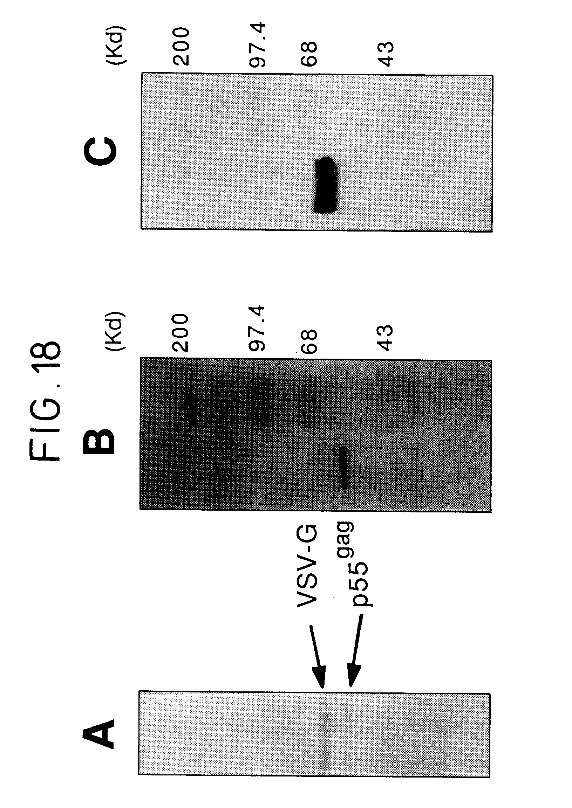

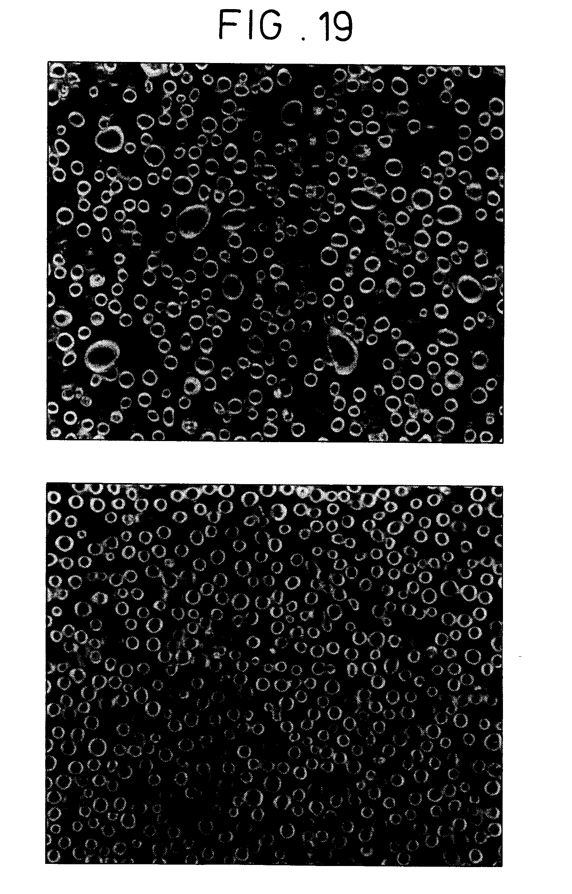

Figure 16 shows that AcOGS740-infected insect cells will bind U937 cells in an adhesion assay at both 37°C and 4°C. No such binding was observed when *Spodoptera frugiperda* cells were infected with the wild-type, AcNPV virus. This result, together with the immunofluorescence, indicates that biologically active VCAM is synthesized and processed in insect cells that are simultaneously expressing gag. *Spodoptera frugiperda* insect cells infected with AcOGS740 will therefore secrete p55$^{gag}$ virus-like particles with biologically active human VCAM incorporated into their lipid envelope, and furthermore, such particles will bind to VLA-4 bearing cells. Such cells include certain antigen presenting cells.

## EXAMPLE 8

The mechanism whereby viral-encoded glycoproteins are picked-up by budding particles is poorly understood. Specific interactions between the transmembrane and/or cytoplasmic domains of the glycoprotein with the cognate capsid protein may be important for efficient incorporation. In consideration of this, the transmembrane and cytoplasmic domains of VCAM were replaced with the corresponding domains from HIV-I gp41 with a view to improving the incorporation of VCAM into budding p55$^{gag}$ particles. The manipulations to construct such a VCAM-gp41 chimaera are summarised in Figure 12 and are described below.

The *Kpn*I fragment encompassing HIV-I gp41 was isolated from the vector pHXB2gpt3' and cloned into the *Kpn*I site of pAcCL29.1 to generate pOGS733.

This placed the *Pst*I site of the vector polylinker downstream of the gp41 gene. A *Pst*I site was introduced by site-directed mutagenesis immediately upstream of the transmembrane domain sequence of gp4 to give pOGS736. Digestion of this plasmid with *Pst*I then allowed the sequence encoding the transmembrane and cytoplasmic domains of gp41 to be isolated.

Site-directed mutagenesis was employed to introduce two new restriction sites into the VCAM gene of pOGS731 to generate pOGS735. These consisted of a *Bam*HI site immediately upstream of the translation initiation codon of VCAM, and a *Pst*I site immediately upstream of the transmembrane domain sequence of VCAM. The VCAM gene was excised from pOGS735 as a *Bam*HI fragment, and cloned into the *Bam*HI site of pAcCL29.1 to generate pOGS739. This placed the *Pst*I site of the vector polylinker immediately downstream of the VCAM gene. Digestion of pOGS739 with *Pst*I therefore released the sequence encoding the transmembrane and cytoplasmic domains of VCAM, which were then replaced with the corresponding gp41 sequence derived from pOGS736 as a *Pst*I figment. This generated the plasmid pOGS742. DNA sequencing has confirmed that the VCAM and gp41 components of this chimaera are in phase, and will thus be translated as a single polypeptide. The VCAM-gp41 hybrid gene was excised from pOGS742 as a *Bam*HI fragment and cloned into the *Bam*HI site of pOGS737 to generate pOGS743. This plasmid thus contains both the HIV-I *gag* and VCAM-gp41 hybrid genes and enables a dual recombinant baculovirus to be constructed which will co-express these two proteins simultaneously. It is expected that the VCAM-gp41 hybrid will be incorporated into *gag* based particles with an efficiency greater than that of the wild-type VCAM molecule. This will increase the avidity of such particles for VLA-4 bearing cells.

## EXAMPLE 9

A recombinant baculovirus expressing the envelope glycoprotein (G protein) of Vesicular Stomatitis Virus (Indiana serotype) was that previously used by Bailey (Bayley, MJ *et al*, 1989, *Virology* 169, 323-331). High titre viral stock of this virus (designated AcVSV-G) were also prepared. VSV-G is a fusogenic protein activated by low pH (Florkiewicz, R & Rose, J, 1984, *Science* 225, 721-723). Cell surface expression of biologically active VSV-G protein in AcVSV-G-infected insect cells was verified by incubation of such cells at pH 5.5. As shown in Figure l7, large multi-nucleated syncytia are formed as a result of VSV-G-mediated cell-cell fusion.

In order for budding p55gag particles to acquire VSV-G protein on their surface, insect cells were co-infected with AcKl-GAG and AcVSV-G such that both proteins were simultaneously expressed in the same cell. This was achieved in the following way. *Spodoptera frugiperda* insect cells were maintained in log growth and grown to a cell density of l.5 x l0⁶/ml before use. Prior to infection, cells were spun down at 900 rpm, 20 ° C, for 8 min, and the culture supernatant discarded. Cells were infected with a mixture of both AcKl-GAG and AcVSV-G such that an moi (multiplicity of infection) of 2pfu (plaque forming units) for each virus per cell was achieved. This ensured that a co-infection was established in the majority of cells with the consequent expression of both foreign proteins. To express p55gag only, cells were infected with AcKl-GAG alone at an moi of 2 pfu per cell. After lh at room temperature, the infected cells were pelleted as before, and the viral inoculum removed. Cells were re-suspended in fresh medium to a density of l.5 x l0⁶ cells/ml and incubated as a suspension culture at 27°C for 66h.

The culture was decanted into sterile 50 ml centrifuge tubes and spun at 3 krpm, 4°C, for l0 min to pellet the cells. p55gag particles were precipitated from the cleared culture supernatant by addition of 5% PEG6000 and mixing at 4°C for 3h. The precipitate was recovered by centrifugation at 9 krpm, 4°C for l0 min, and re-suspended in TEN to l/l0th the original culture volume. After a 5 min spin at 3 krpm, 4°C, to pellet aggregated material, the re-suspended PEG precipitate was loaded onto linear sucrose gradients (l0-50% sucrose in TEN:l0mM Tris-HCl pH 7.4, lmM EDTA, l50mM NaCL) and centrifuged at l97,000g, l0°C for 20 min. Under these centrifugal forces monomeric VSV-G protein does not sediment significantly through the gradient. By contrast, VSV-G protein associated with particles will have a greatly increased apparent sedimentation coefficient and will therefore sediment through the gradient. After centrifugation, the gradient was fractionated and the p55gag particles recovered. Figure l8 shows both p55gag and VSV-G protein to be present when this material was resolved by SDS-PAGE demonstrating their co-purification. Confirmation of the authenticity of these proteins was provided by Western blot with an anti-p24 monoclonal antibody and an anti-VSV antibody (see Figure l8).

An alternative, simpler, purification procedure was adopted to isolate material for assessment of the fusogenic activity of VSV-G protein incorporated into p55gag particles. This consisted of co-infecting insect cells with AcKl-GAG and AcVSV-G (*or AcKl-GAG alone as control*) and harvesting as described, then loading the cleared culture supernatant onto sucrose step gradients (60% sucrose overlayered with 30 % sucrose both in TEN and centrifugation at 96,000g, 4°C for 2h. Particles were recovered from the 30/60% sucrose interface, and after pooling of the material from several gradients, further concentrated on a similar sucrose step gradient. Following recovery, the samples were dialysed overnight against l0mM HEPES pH7.4, l50mM NaCl at 4°C to remove the sucrose.

The fusogenic activity of VSV-G protein incorporated into the envelope of p55gag particles was initially assess by the ability of these particles to induce syncytia formation after their adsorption to *Spodoptera frugiperda* insect cells. 35 mm tissue culture dishes were seeded with l.5 x l0⁶ insect cells each, and after attachment of the cells to the dish, were cooled to 4°C. The culture supernatant was discarded and the cells washed twice with l45 mM NaCl, l0 mM HEPES pH 7.4, before addition of 250μl of p55gag virus-like particles or p55gag virus-like particles presenting VSV-G (also cooled to 4°C). Following a lh adsorption at 4°C, the particles were removed and replaced with 2 ml of tissue culture medium pH 5.5 at 28°C for l min. This low pH medium triggers the fusogenic activity of VSV-G. This medium was then removed and replaced with 2 ml of tissue culture medium at standard pH (6. 2) and incubated at 28 °C for 2h. Figure l9 shows that numerous syncytia had developed in the cells to which p55gag particles purified from cells co-expressing VSV-G protein had been adsorbed. No syncytia had formed between cells after exposure to p55gag particles alone. This demonstrates that VSV-G protein incorporated into budding particles is fusogenically active after exposure to low pH, and that this activity is retained aft purification procedures.

To confirm further that VSV-G protein incorporated into budding particles is biologically functional, the haemolytic activity of p55gag particles presenting VSV-G was determined. VSV has been demonstrated to possess an acid pH-dependent haemolytic activity (Baily, C *et al*, l98l, *J. Cell Biol.* **9l**, llla) mediated by the G protein (Schlegel, R. & Wade, M. l984, *J. Biol. Chem.* **259**, 469l-4694). Haemolytic activity was measured by the following assay. Sheep red blood cells (supplied as l0% in alseviers; Serotec, UK) were pelleted by centrifugation at 250g, 4°C for 5 min, and washed twice in PBS. The cells were divided into two aliquots and washed with haemolysis buffer (l0mM MES, l50mM NaCl) at pH 5.0 or pH 7.0. After final centrifugation, the cells were re-suspended to a concentration of l0% (v/v) in haemolysis buffer of the same pH. l00μl of particles isolated by the above procedure (containing approx. 20μg p55gag) were incubated with 500μl of l0 % sheep red blood cells at either pH 5.0 or 7.0 for 20min at 37°C. As a control, l00μl of l0mM HEPES pH 7.4, l50mM NaCl was included to estimate the spontaneous haemolysis of red blood cells at each pH. The cells were then pelleted by centrifugation as described, and the optical density at 54lnm taken as an estimation of the haemoglobin concentration in the supernatant. As shown in Table l, p55gag particles alone have no significant haemolytic activity, however, those purified from insect cells co-expressing VSV-G protein possessed a high, pH-dependent activity.

TABLE I shows the release of haemoglobin from sheep red blood cells revealed by the increase in optical density at 541nm following VSV-G mediated haemolysis. A marked acid pH-dependent haemolytic activity of p55$^{gag}$/VSV-G particles, but not p55$^{gag}$ particles, is demonstrated. Values are expressed as $OD_{541}$ units per 100μl sample (approx. 20μg p55$_{gag}$) and are adjusted to exclude spontaneous haemolysis by subtraction of the control sample OD at the designated pH.

|  | HAEMOLYSIS BUFFER | |
|---|---|---|
| SAMPLE | pH 5.0 | pH 7.0 |
| p55$^{gag}$ particles | 0.18 | 0.44 |
| p55$^{gag}$/VSV-G particles | 4.61 | 0.69 |

## EXAMPLE 10

In the previously described examples the self-assembling protein is the same as the naturally occurring sequence of HIV-I GAG. However in the described invention this need not be the case. The self assembling molecule may be truncated and/or supplemented by one or more additional amino acids. This heterologous sequence may have additional biological activity. Such a heterologous sequence could be any amino acid sequence either known to the art or yet to be elucidated. The biological activity may be a single type of activity (for example, immunogenicity, receptor-binding activity, therapeutic activity or targeting activity), or it may have a combination of two or more different biological activities. The heterologous amino acid sequence may be an antigen of an infectious agent. In the example below, the heterologous sequence used is the third variable domain, or GPGR loop sequence, found between amino acids 300 and 330 of the envelope glycoprotein gpl20 of HIV-I. The GPGR loop is defined by two flanking cysteine residues linked by a disulphide bond, and is the major neutralizing epitope for HIV-I (Putney *et al* 1986 *Science* **234**, 1392; Rusche *et al* 1988 *Proc. Natl. Acad. Sci.* **85**, 3198; Palker *et al* 1988 *Proc. Natl. Acad. Sci.* **85**, 1932; Goudsmit *et al* 1988 *AIDS* **2**, 157).

This example describes the construction of hybrid HIV-I GAG particles containing the GPGR loop from the HIV-I envelope glycoprotein gpl20, with reference to certain figures of the drawings.

Recombinant DNA manipulations were as described in Example I.

Oligonucleotides were synthesized by automated phosphoramidite chemistry using cyanoethyl phosphoramidites (Beaucage and Caruthers 1981 *Tetrahedron Letters* **24**, 245). Following de-blocking and removal from the controlled pore glass support the oligomers were purified on denaturing polyacrylamide gels, further purified by ethanol precipitation and finally dissolved in water prior to estimation of their concentration. The oligomers were then kinased to provide them with a 5′ phosphate as required for the ligation step. Complementary oligomers were then annealed prior to ligation into the relevant plasmid vector. The sequence of the synthetic oligomers was confirmed by dideoxy sequencing. The protocol used was essentially as has been described (Biggin *et al* 1983 *Proc. Natl. Acad. Sci.* **80**, 3963) and modified to allow sequencing on plasmid DNA as described (Guo and Wu 1983 *Nucl. Acid. Res.* **II**, 5521).

HIV-I *gag* constructs were derived from pOGS2 as described in Example I.

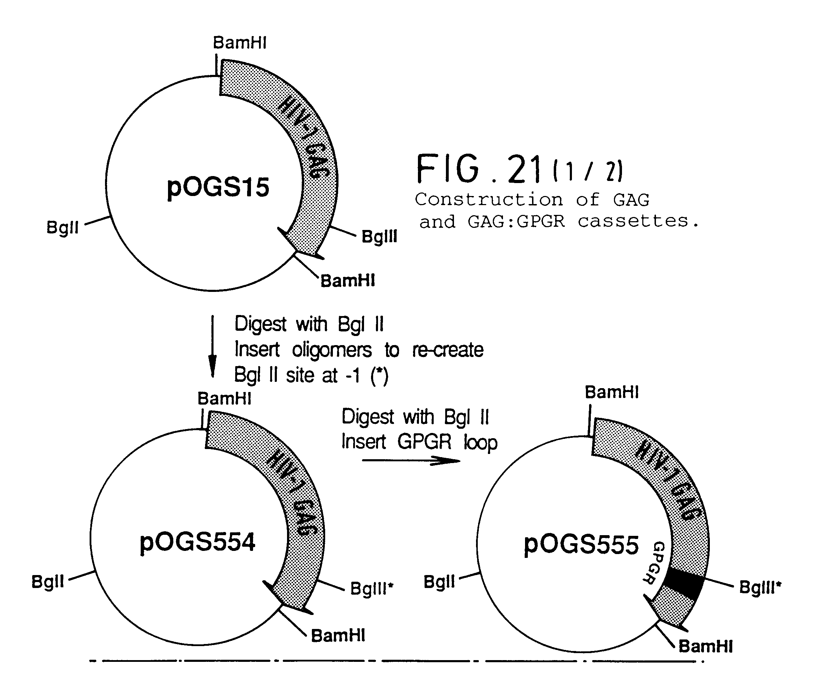

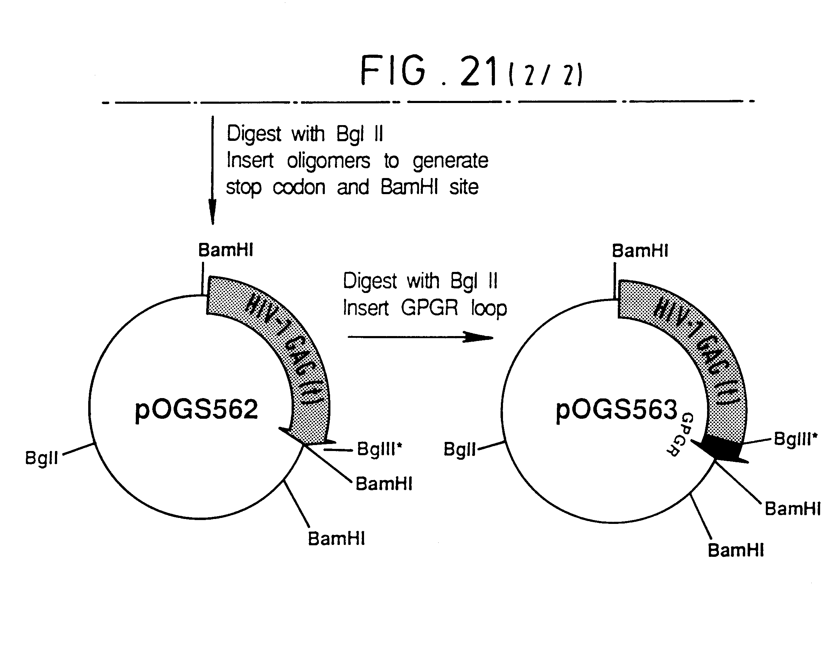

A fusion gene containing the whole of HIV-I *gag* and a GPGR loop sequence was made by inserting synthetic oligomers encoding the GPGR loop sequence from HIV-I isolate HXB2 (Fisher *et al* 1986 *Science* **233**, 655) at the *Bgl*II site and ligated with synthetic oligomers SEAGAG/03 and SEAGAG/04 (Figure 20). This insertion re-creates a *Bgl*II site-I with respect to the original *Bgl*II site within the *gag* gene. The resulting plasmid was designated pOGS554. Synthetic oligomers encoding the HXB2 GPGR loop sequence were inserted into pOGS554 that had been cleaved at the new *Bgl*II site. This generated plasmid pOGS555 which contains the *gag* (full-length): GPGR loop fusion gene as a *Bam*HI cassette (Figure 21).

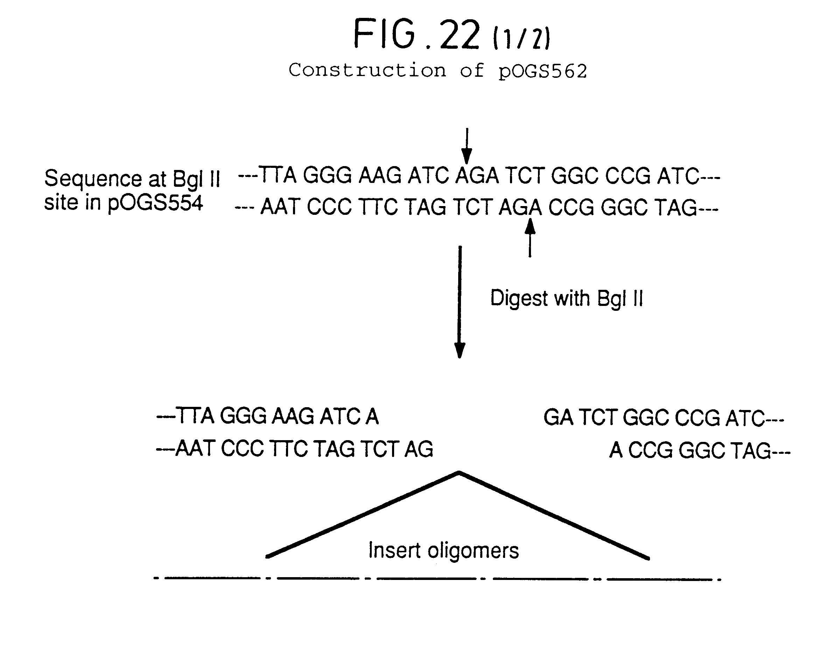

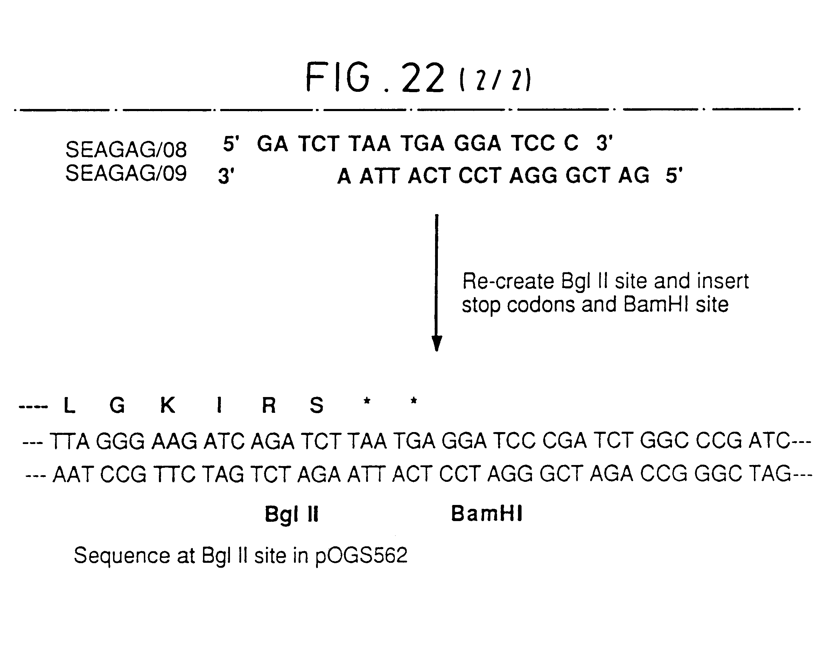

In order to produce a *gag* gene in which the C-terminal 75 residues have been removed, synthetic oligomers SEAGAG/08 and SEAGAG/09 were inserted into pOGS554 which had been cleaved with *Bgl*II. This insertion results in the generation of a stop codon and a *Bam*HI site down stream of the *Bal*I site. The resulting plasmid was designated pOGS562 and contains a *gag*(truncated):GPGR loop fusion gene as a *Bam*HI cassette (Figures 2l and 22).

A fusion gene containing the truncated HIV-I gag gene and a GPGR loop sequence was made by inserting synthetic oligomers encoding the GPGR loop sequence from HIV-I isolate HXB2 (Fisher *et al* l986 *op cit*) at the *Bgl*II site in plasmid pOGS562. The resulting plasmid was designated pOGS563 and contains the *gag* (truncated): GPGR loop fusion gene as a *Bam*HI cassette (Figure 2l).

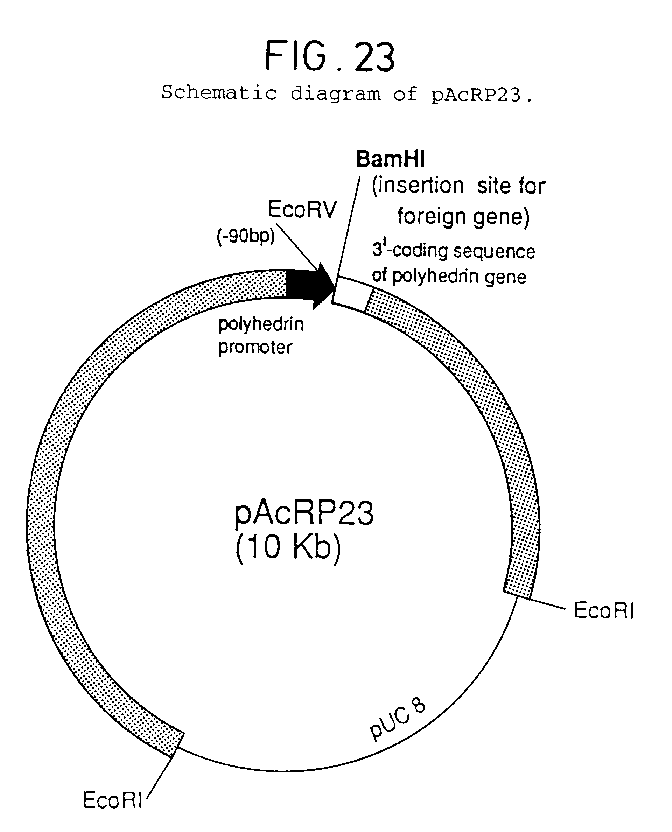

Each of the four *gag* cassettes (full-length *gag*; full-length *gag*/GPGR loop; truncated *gag*; truncated *gag*/GPGR loop) were excised from pOGSl5, pOGS555, pOGS562 and pOGS563 as *Bam*HI fragments and cloned into the baculovirus expression vector pAcRP23 (Figure 23) to generate pAcKlgag, pOGS556, pOGS572 and pOGS574 respectively. Plasmid pAcRP23 contains the powerful polyhedrin promoter from *Autographa californica* nuclear polyhedrosis virus (AcNPV) and a unique *Bam*HI site for insertion of foreign genes flanked by AcPNV sequences. Plasmid pAcRP23 also contains sequences for replication and selection of the plasmid in *E. coli* (Matsuura *et al* l987 *J. Gen. Virol.* **68**, l233). When cloned into the *Bam*HI site of this vector, the *gag* gene derivatives come under the control of the polyhedrin promoter (Figure 23).

The cell line designated *Spodoptera frugiperda* 9 (Sf9) (Summers and Smith l987 Texas Agricultural Experiment Station Bulletin No. l555), which is a clonal derivative of the lepidopteran line IPLB-Sf2l-AE (Vaughn *et al* l977 *In Vitro* l3, 2l3), was used for all experiments. Cells were grown in suspension culture in medium comprising TCl00 (GIBCO-BRL, UK) containing l0% heat inactivated fetal calf serum (ICN-FLOW Laboratories, UK) plus 50IU/ml penicillin and 50mg/ml streptomycin (GIBCO-BRL, UK).

Recombinant baculoviruses were prepared by utilizing the phenomenon of *in vivo* recombination between homologous DNA sequences. The viral (AcNPV) DNA and plasmid DNA (pAcKlgag, pOGS556, pOGS572 or pOGS574) were introduced into SF9 insect cells by the method of calcium chloride precipitation and progeny were recovered after 48 hours (Summers and Smith, *op cit*). Recombinant baculoviruses were selected on the basis of a polyhedrin-negative phenotype in a Brown & Faulkner plaque assay (Brown and Faulkner l977 *J. Gen. Virol.* **36**, 36l). After plaque purification, high titre viral stocks of the recombinant viruses were prepared and their titres determined in plaque assays. Titres were in excess of $5 \times 10^7$ plaque forming units (pfu) per ml. Recombinant viruses were designated AcKlgag (full-length *gag*), AcOGS556 (full-length *gag*/GPGR loop), AcOGS572 (truncated *gag*) and AcOGS574 (truncated *gag*/GPGR loop).

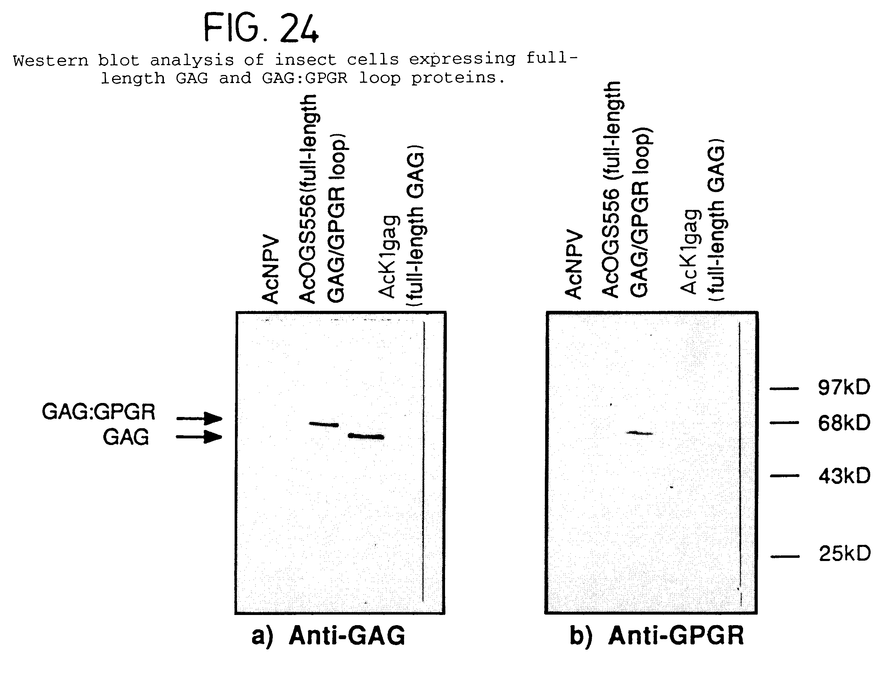

Expression of the full-length GAG and full-length GAG/GPGR loop proteins was examined by Western blot analysis. Confluent monolayers of SF9 cells were infected with the recombinant virus AcKlgag, recombinant virus AcOGS556 or wild-type AcNPV at a multiplicity of infection of 5pfu per cell. The cells were harvested at 48 hours post-infection and pelleted by centrifugation at l000g. After washing with phosphate-buffered saline (PBS), the cells were lysed and the proteins resolved by SDS-PAGE as described by Laemmli (Laemmli l970 *Nature* **227**, 68). The separated proteins were transferred to nitrocellulose as described by Towbin (Towbin *et al* l979 *Proc. Natl. Acad. Sci.* **76**, 4350) and probes with either anti-GAG or anti-GPGR monoclonal antibodies (MAb; Du Pont-UK). Cells infected with AcKl gag contained a protein of approximately 55kD which reacted with the anti-GAG MAb but not with the anti-GPGR MAb. In contrast, cells infected with AcOGS556 contained a protein of approximately 59kD which reacted with both the anti-GAG and anti-GPGR MAbs, confirming the expression of the full-length GAG/GPGR loop fusion gene as a fusion protein (Figure 24).

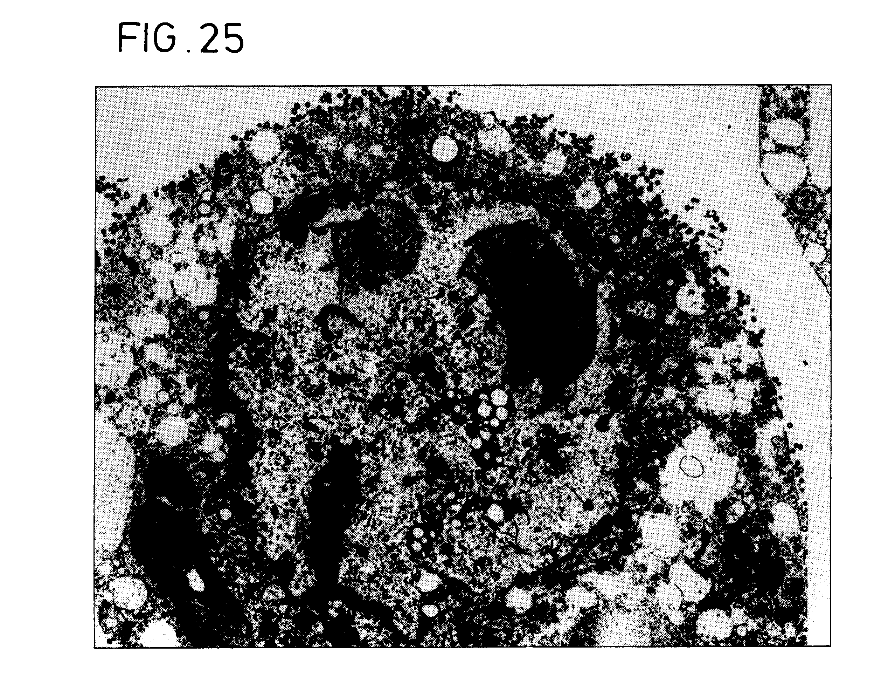

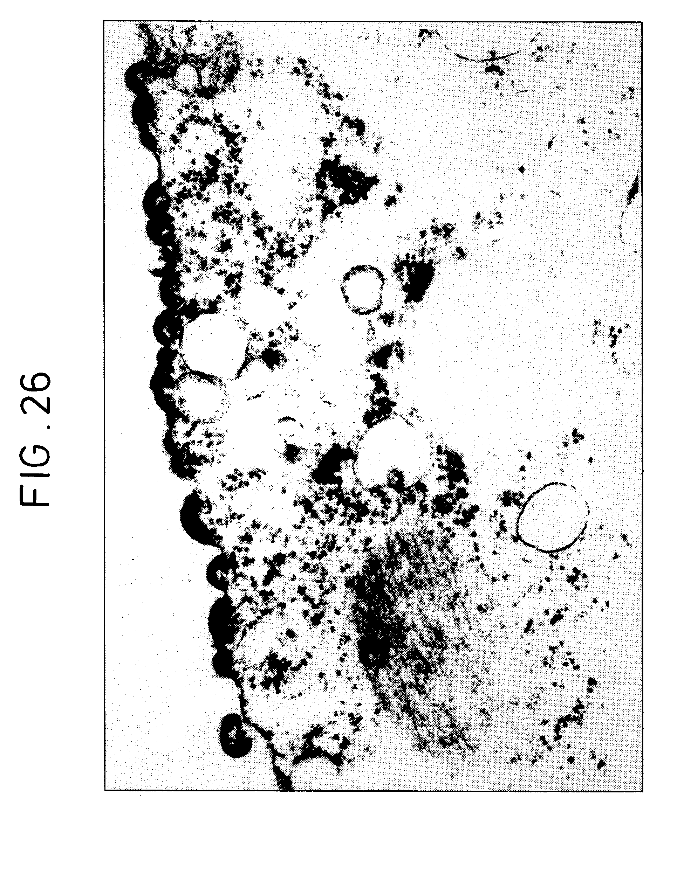

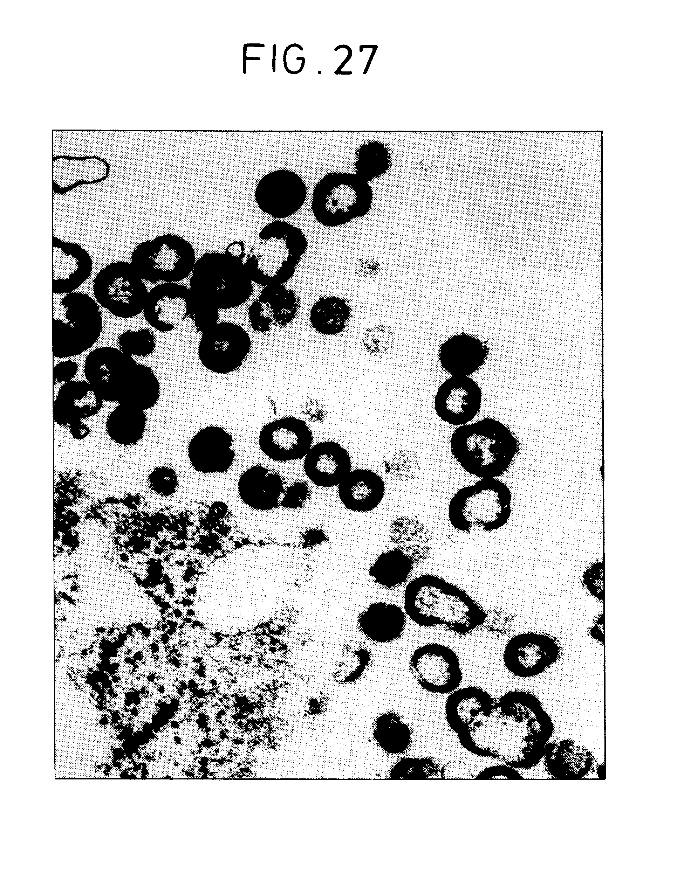

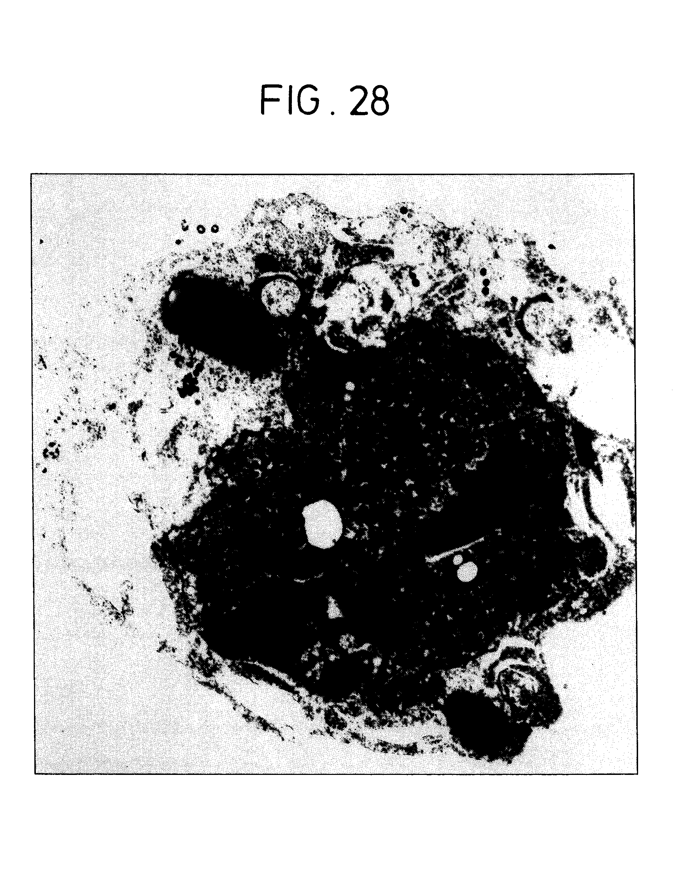

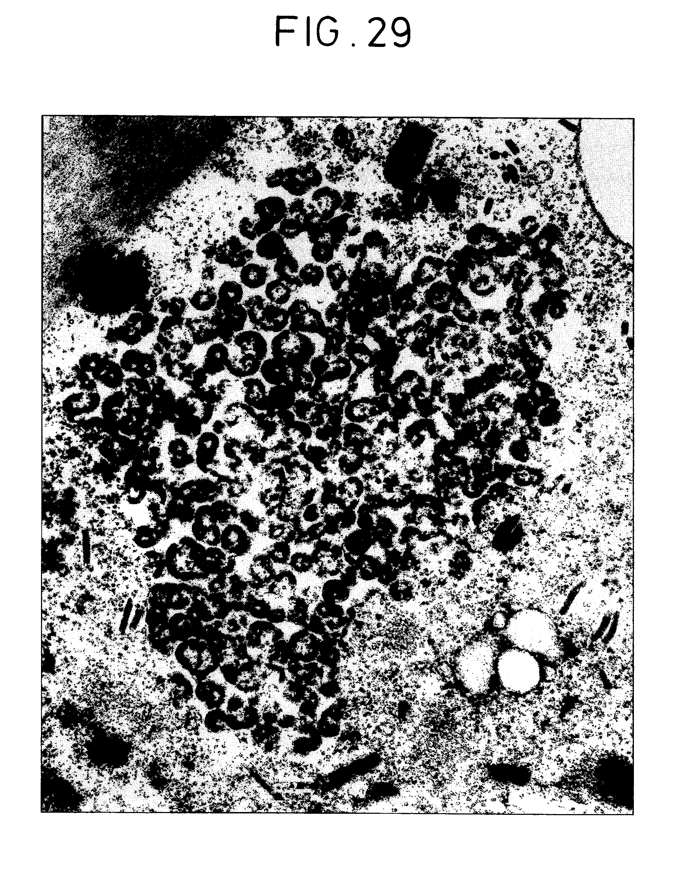

When thin sections of cells infected with AcKlgag (full-length GAG) were examined using electron microscopy, virus-like particles could be seen budding from the plasma membrane (Figures 25 and 26). Figure 27 demonstrates that the budded particles showed some irregularity, although they had a similar morphology to immature HIV virions as has been observed previously by Gheyson (Gheyson *et al* l989 *Cell* **59**, l03). In contrast, electron microscopic analysis of cells infected with AcOGS556 (full-length GAG/GPGR loop) showed that the hybrid protein accumulated as largely aggregated material in the nucleus (Figure 28). In some instances partially spherical structures were evident, which although predominantly irregular, occasionally had the appearance of virus-like particles (Figure 29). These results suggested that it may be possible to generate hybrid GAG particles, but that modification of the particle-forming sequence would be necessary for the formation of regular, stable structures.

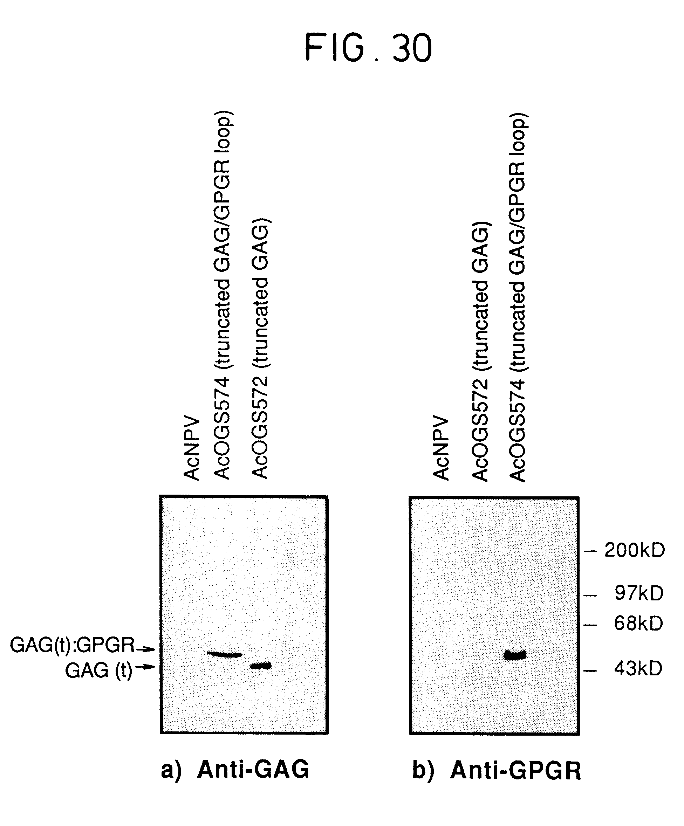

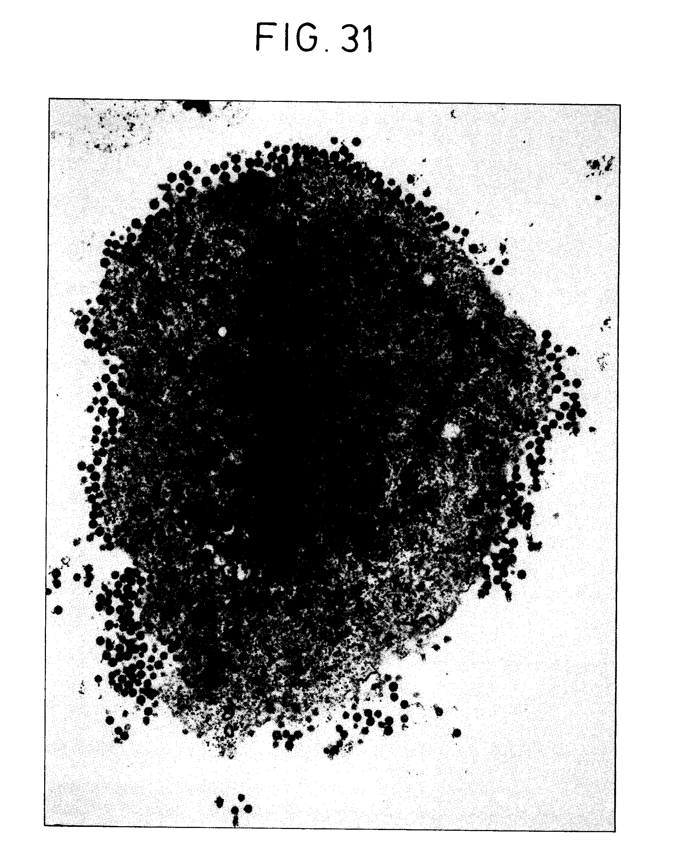

Expression of the truncated GAG and truncated GAG/GPGR loop proteins was also examined by Western blot analysis. Confluent monolayers of SF9 cells were infected with the recombinant virus AcOGS572, recombinant virus AcOGS574 or wild-type AcNPV at multiplicity of infection of 5pfu per cell. The cells were harvested, lysed and the proteins separated as described above. As expected, cells infected with AcOGS572 contained a protein of approximately 47kD which reacted with the anti-GAG MAb but not with the anti-GPGR MAb. Cells infected with AcOGS574 contained a protein of approximately 54kD which reacted with both the anti-GAG and anti-GPGR MAbs, confirming the expression of the truncated GAG/GPGR loop fusion gene as a fusion protein (Figure 30).

Electron microscopic analysis of cells infected with AcOGS572 demonstrated the presence of virus-like particles budding from the plasma membrane (Figures 3l and 32). In contrast to the virus-like particles budding from cells infected with AcKlgag (full-length GAG), the truncated GAG particles appeared to be more regular (Figure 33). Even more striking was the appearance of regular particles, mainly in the nucleus, of cells infected with AcOGS574 (truncated GAG/GPGR loop; Figures 34 and 35). In addition, some of the hybrid truncated GAG:GPGR loop particles could be seen budding from the cell surface (Figure 36). These data demonstrate that the formation of hybrid GAG particles from *gag* fusion genes is dependent on modification of the *gag* gene. Furthermore the data demonstrate that the first 437 amino acids of the HIV-I GAG protein contain sufficient information to form virus-like particles.

It is anticipated that, as exemplified in Examples 2 to 9, the expression of the truncated GAG or the truncated GAG/GPGR loop proteins in the same cell and a heterologous membrane bound protein, of natural or modified sequence, will result in the membrane bound protein being incorporated into particles that bud from membranes. In so doing, the budded particles will acquire some, if not all, of the properties of the membrane bound protein. These properties include antigenicity, targeting and pharmacological activities. The expression of the two proteins may be from one or more vectors and in any cell type of animal origin.

## Claims

1. A proteinaceous, lipid-containing particle comprising a budding assembly formed from a plurality of self-assembling protein moieties, the assembly being at least partially enveloped with a lipid bilayer and one or more membrane-bound moieties, heterologous with respect to the self-assembling protein moieties, wherein, if the self-assembling protein moieties are of viral origin, the particle is free of infectious viral nucleic acid.

2. A particle as claimed in claim I, wherein the self- assembling protein moieties are of viral origin or related to proteins of viral origin.

3. A particle as claimed in claim 2, wherein the self- assembling protein is a GAG protein of a retrovirus.

4. A particle as claimed in claim 3, wherein the retrovirus is an Human Immunodeficiency Virus (HIV), a T-lymphocyte virus, Simian Immunodeficiency Virus (SIV) or Bovine Immunodeficiency-like virus (BIV).

5. A particle as claimed in claim 4, wherein the self- assembling protein moieties are budding core molecules of Human Immunodeficiency Virus I (HIV-I).

6. A particle as claimed in any one of claims I to 5, wherein the nucleic acid binding ability of the self- assembling protein is modified compared to a natural protein from which it is derived.

7. A particle as claimed in any one of claims I to 6, wherein the self-assembling protein moiety comprises a first amino acid sequence and a second amino acid sequence, wherein the first amino acid sequence confers on the protein molecule its self-assembling property and the second amino acid sequence confers a desired biological activity.

8. A particle as claimed in claim 7, wherein the biological activity of the second amino acid sequence is antigenicity, enzymic activity and/or cytotoxic activity and/or nucleic acid-binding activity.

9. A particle as claimed in claim 8, wherein the second amino acid sequence is substantially homologous to the third viable domain of HIV-I gpl20.

10. A particle as claimed in claim 8, wherein the second amino acid sequence is capable of binding to a ribozyme, antisense RNA, DNA or messenger RNA.

11. A particle as claimed in any one of claims I to I0, wherein the self-assembling protein moiety, or a self-assembling part of it, has at least 70% homology with a natural protein.

12. A particle as claimed in any one of claims I to II, wherein the lipid bilayer is derived from a cell in which the assembly has been put together and/or in which the synthesis of the self-assembling moieties has taken place.

13. A particle as claimed in any one of claims I to I2, wherein the lipid substantially completely envelopes the core assembly.

14. A particle as claimed in any one of claims I to I3 wherein the membrane-bound protein moiety(ies) confer(s) additional biological activity in the particle.

15. A particle as claimed in claim I4, wherein the membrane-bound moiety comprises a plasma membrane localisation and/or retention signal.

16. A particle as claimed in claim I4 or I5, wherein the membrane-bound moiety confers an immunogenic ability, a site-specific targeting ability, fusogenic properties, binding or packaging activity, enzymic activity, cytotoxicity and/or pharmaceutical activity.

17. A particle as claimed in claim I4 or I5, wherein the membrane-bound moiety either is, or includes sequences from, a viral protein.

18. A particle as claimed in claim I7, wherein the viral protein is the influenza virus haemagglutinin protein or the env-derived proteins of a retrovirus.

19. A particle as claimed in claim I4 or I5, wherein the membrane bound moiety confers upon the particle the property of binding to cells of the immune system.

20. A particle as claimed in claim I9, wherein the membrane bound moiety has the activity of CD4, CD8, ICAM-I, ICAM-2 or VCAM or BBI.

21. A particle as claimed in claim I4 or I5, wherein the membrane-bound moiety has the fusogenic property of HIV-I gp4I, VSV-G, influenza haemagglutinin, Sendai Virus fusion factor (F), or a glycoprotein of Semliki Forest Virus.

22. A particle as claimed in claim I4 or I5, wherein the membrane-bound moiety has T cell proliferative activity.

23. A process for the preparation of a proteinaceous, lipid-containing particle, the process comprising expressing in a host cell protein moieties which are capable of self-assembly, and wherein the resulting assembly is capable of budding off from the host cell thereby to acquire an at least partial envelope of lipid.

24. A process as claimed in claim 23, wherein a membrane-bound protein moiety is co-expressed in the same host cell as the self-assembling protein.

25. A process as claimed in claim 23 or 24, wherein expression takes place in mammalian or insect cells.

26. A process as claimed in claim 25, wherein expression takes place in an insect cell by means of a baculovirus expression system.

27. An animal cell expression vector comprising first and second coding sequences, wherein the first coding sequence encodes a protein moiety which is capable of self- assembling into an assembly of proteins which is in turn capable of budding off from an animal cell to form a lipid-enveloped particle, and wherein the second coding sequence encodes a membrane-bound protein moiety, the coding sequences being operatively linked, optionally respectively, to a heterologous promoter.

28. A set of animal cell expression vectors there is provided a set of animal cell expression vectors, the set comprising:
(a) a first vector having a coding sequence encoding a protein moiety which is capable of self- assembling into an assembly of proteins which is in turn capable of budding off from an animal cell to form a lipid-enveloped particle, the coding sequence being operatively linked to a heterologous promoter; and
(b) a second vector having a coding sequence encoding a membrane-bound protein moiety, the coding sequence being operatively linked to a heterologous promoter.

29. A host cell comprising an expression vector as claimed in claim 27 or a set of expression vectors as claimed in claim 28.

30. A pharmaceutical or diagnostic composition comprising particles as claimed in any one of claims I to 22 and a suitable carrier.

31. A pharmaceutical composition as claimed in claim 30 which is a vaccine and which is sterile.

32. A detectably labelled particle as claimed in any one of claims I to 22.

19

# FIG . 1
## Construction of HIV-1 GAG cassette

a)    b)

FIG. 2

p55 $^{gag}$ →

FIG. 4

← Influenza haemagglutinin

FIG . 3

FIG. 5

FIG. 7

# FIG . 6

a)          b)

p55$^{gag}$ →          ← Influenza haemagglutinin

# FIG . 8

a)      b)

Dilution
Factor

1:256

1:512

1:1,024

1:2,048

1:4,096

1:8,192

1:16,384

# FIG . 9

GAG                    GAG + HA

u        p        s        u        p        s

p55 $^{GAG}$ →

# FIG . 10

1        2        Marker        (Kd)

200

97.4

p55 $^{gag}$ →        68

43

29

## FIG . 11

## FIG . 13

EP 0 508 809 A1

pCDM8.VCAM  ⟶  VCAM + pUC18

**pOGS723**

   PstI-BamHI
   digestion

VCAM + pAcCL29.8

**pOGS731**

# FIG.12 (1/3)

BamHI site
upstream ATG

BamHI site upstream ATG
plus PstI site upstream
TM domain

MUTAGENESIS

pHXB2gpt3'

KpnI digestion

pOGS15

| BamHI
| digestion
↓

GAG  +  pAcUW51

↓

**pOGS737** +  VCAM

**pOGS734**

| partial BamHI
| digestion
↓

VCAM

pAcYM1

**pOGS738**

**pOGS735**

| partial BamHI
| digestion
↓

pAcCL29.1  +  VCAM

↓

**pOGS739**

pAcCL29.1 + gp41

↓

**pOGS733**

| MUTAGENESIS
| PstI site upstream
| TM domain
↓

**pOGS736**

EP 0 508 809 A1

pOGS740

Vector containing EC
domain VCAM    +    TM and CYT domains
gp41

PstI digestion,
isolate vector

PstI digestion,
isolate fragment

**pOGS742**

Multiple partial
BamHI digestion

**pOGS737** +  VCAM-gp41 hybrid

pOGS743

EP 0 508 809 A1

# FIG . 14

# FIG .15

# FIG . 16

# FIG.17

# FIG. 18

**A**

**B** (Kd)

**C** (Kd)

VSV-G ←

p55$^{gag}$ ←

200

97.4

68

43

200

97.4

68

43

EP 0 508 809 A1

# FIG . 19

# FIG . 20
Construction of pOGS554.

**Bgl II**

Sequence at Bgl II
site in pOGS15

---TTA GGG AAG ATC TGG CCT TCC---
---AAT CCC TTC TAG ACC GGA AGG---

Digest with Bgl II

---TTA GGG AA             G ATC TGG CCT TCC---
---AAT CCC TTC TAG             ACC GGA AGG--

Insert oligomers

SEAGAG/03      5' G ATC **AGA TCT** GGC CC 3'
SEAGAG/04      3'          **TCT AGA** CCG GGC TAG 5'

Re-create Bgl II site at -1 with
respect to original site

---TTA GGG AAG ATC  **AGA TCT**  GGC CCG ATC TGG CCT TCC---
---AAT CCC TTC TAG  **TCT AGA**  CCG GGC TAG ACC GGA AGG---

Sequence at new Bgl II site in pOGS554

FIG.21 (1/2)
Construction of GAG and GAG:GPGR cassettes.

Digest with Bgl II
Insert oligomers to generate
stop codon and BamHI site

Digest with Bgl II
Insert GPGR loop

BamHI

HIV-1 GAG (1)

pOGS562

BgII

BglII*

BamHI

BamHI

BamHI

HIV-1 GAG (1)

pOGS563

GPGR

BgII

BglII*

BamHI

BamHI

EP 0 508 809 A1

Construction of pOGS562

Sequence at Bgl II ---TTA GGG AAG ATC AGA TCT GGC CCG ATC---
site in pOGS554 --- AAT CCC TTC TAG TCT AGA CCG GGC TAG---

Digest with Bgl II

—TTA GGG AAG ATC A                    GA TCT GGC CCG ATC---
—AAT CCC TTC TAG TCT AG                    A CCG GGC TAG---

Insert oligomers

EP 0 508 809 A1

SEAGAG/08 5' GA TCT TAA TGA GGA TCC C 3'
SEAGAG/09 3'    A ATT ACT CCT AGG GCT AG 5'

Re-create Bgl II site and insert
stop codons and BamHI site

```
---  L    G    K    I    R    S    *    *
--- TTA GGG AAG ATC AGA TCT TAA TGA GGA TCC CGA TCT GGC CCG ATC---
--- AAT CCG TTC TAG TCT AGA ATT ACT CCT AGG GCT AGA CCG GGC TAG---
```

    **Bgl II**    **BamHI**

Sequence at Bgl II site in pOGS562

EP 0 508 809 A1

# FIG . 23

Schematic diagram of pAcRP23.

**BamHI**
(insertion site for
foreign gene)

3'-coding sequence
of polyhedrin gene

EcoRV

(-90bp)

polyhedrin
promoter

pAcRP23
(10 Kb)

EcoRI

pUC 8

EcoRI

# FIG. 24

Western blot analysis of insect cells expressing full-length GAG and GAG:GPGR loop proteins.

a) **Anti-GAG**   b) **Anti-GPGR**

EP 0 508 809 A1

FIG. 25

FIG . 26

# FIG . 27

FIG . 28

# FIG . 29

# FIG. 30

a) **Anti-GAG**    b) **Anti-GPGR**

47

# FIG. 31

FIG . 32

# FIG.33

FIG . 34

# FIG . 35

# FIG . 36

European Patent
Office

**EUROPEAN SEARCH REPORT**

Application Number

EP    92 30 3223

Page 1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| D,X | WO-A-8 803 562 (OXFORD GENE SYSTEMS LIMITED)<br><br>* the whole document *<br>--- | 1--5,<br>7-9,<br>11--19,<br>21-25,<br>27,29-31 | C12N7/04<br>C12N15/86<br>C12N15/12<br>C12N15/41<br>C12N15/47<br>C12N15/49<br>C12N15/87<br>C12N5/10<br>C12N15/85<br>A61K37/00 |
| D,X | WO-A-8 803 563 (OXFORD GENE SYSTEMS LIMITED)<br><br>* the whole document *<br>--- | 1-5,78,<br>11,12,<br>14,16,<br>17,21,<br>23-25,<br>27,29-31 | |
| X | EP-A-0 415 731 (THE WELLCOME FOUNDATION)<br><br>* the whole document *<br>--- | 1-3,7,8,<br>11-17,<br>23-25,<br>27-31 | |
| X | EP-A-0 398 332 (RESEARCH CORPORATION)<br><br>* the whole document *<br>--- | 1-3,<br>6--8,11,<br>16,<br>23-25,<br>27,29-31 | TECHNICAL FIELDS<br>SEARCHED (Int. Cl.5 )<br><br>C12N<br>C07K |
| X | EP-A-0 334 301 (VIAGENE)<br><br>* the whole document *<br>---<br><br>-/-- | 1-5,7-9,<br>11-21,<br>23-25,<br>27-31 | |

The present search report has been drawn up for all claims

| Place of search | Date of completion of the search | Examiner |
|---|---|---|
| THE HAGUE | 01 JULY 1992 | CHAMBONNET F.J. |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons
................................................................................
& : member of the same patent family, corresponding
    document

EPO FORM 1503 03.82 (P0401)

**European Patent Office**

**EUROPEAN SEARCH REPORT**

Application Number

EP    92 30 3223

Page 2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | EP-A-0 386 882 (DANA FARBER CANCER INSTITUTE)<br><br><br>* the whole document * <br>--- | 1-5,8,9,<br>11-14,<br>16-19,<br>21,<br>23-25,<br>27,29-31 | |
| X | JOURNAL OF VIROLOGY<br>vol. 65, no. 3, March 1991,<br>pages 1202 - 1207;<br>EMI, N. ET AL.: 'Pseudotype formation of Murine Leukemia Virus with the G protein of Vesicular Stomatitis Virus'<br>* the whole document *<br><br>----- | 1-4,7,8,<br>11-114,<br>16,17,<br>23-25,<br>27,29-31 | |

TECHNICAL FIELDS
SEARCHED (Int. Cl.5)

The present search report has been drawn up for all claims

| Place of search | Date of completion of the search | Examiner |
|---|---|---|
| THE HAGUE | 01 JULY 1992 | CHAMBONNET F.J. |